# FASCICULE DE BREVET EUROPEEN

(11) **EP 2 208 510 B1**
(45) Date de publication et mention de la délivrance du brevet: **30.03.2011**
(21) Numéro de dépôt: 10150561.8
(22) Date de dépôt: 12.01.2010
(51) Int. Cl.: A61Q 19/08, A61K 8/81, A61K 8/67, A61K 8/60

(54) **Composition cosmétique ou dermatologique, comprenant un rétinoïde, un composé non phosphaté à base d'adénosine et un polymère semi-cristallin**
Kosmetische oder dermatologische Zusammensetzung, die ein Retinoid, eine nicht-phosphatierte Verbindung auf der Basis von Adenosin und ein halbkristallines Polymer enthält
Cosmetic or dermatological composition comprising a retinoid, a non-phosphated compound based on adenosine and a semi-crystalline polymer

(30) Priorité: 15.01.2009 FR 0950238; 03.02.2009 US 149346 P
(43) Date de publication de la demande: 21.07.2010
(73) Titulaire: L'Oréal, 75008 Paris (FR)
(72) Inventeur: Ravaux, Danielle, 78730, SAINT-ARNOULT EN YVELINES (FR); Fonolla Moreno, Angeles, 75013, PARIS (FR)
(74) Mandataire: Le Coupanec, Pascale A.M.P.

(56) Documents cités:
- EP-A- 1 647 257
- EP-A- 1 847 547
- EP-A- 1 961 455
- WO-A-2007/141142
- DATABASE WPI Week 199812 Thomson Scientific, London, GB; AN 1998-126067 XP002547506 & JP 10 007541 A (NOEVIR KK) 13 janvier 1998 (1998-01-13)

## Description

La présente invention concerne des compositions cosmétiques ou dermatologiques comprenant une association d'actifs destinée à lutter contre les signes cutanés du vieillissement, et notamment contre la peau ridée et/ou la peau relâchée.

Le souci de conserver le plus longtemps possible une peau d'aspect jeune est une préoccupation de la majorité des femmes et concerne également de plus en plus les hommes. Pour répondre à cette attente, des compositions cosmétiques visant la prévention et/ou le traitement des signes de vieillissement cutané ont donc été développées.

Le vieillissement cutané est défini par l'ensemble des altérations du revêtement cutané, résultant de l'accumulation au fil des années des modifications progressives de ses différents constituants.

Il se traduit entre autre par un aplatissement de l'épiderme et de la jonction dermo-épidermique et au niveau du derme, par une réduction globale de la matrice extracellulaire (MEC) associée à une diminution progressive de la production des fibres de collagène et d'élastine par les fibroblastes d'une part et d'autre part une augmentation de la destruction de ces macromolécules par des enzymes spécifiques.

Ces phénomènes biologiques ont donc pour conséquence d'induire au niveau de la peau d'importantes modifications physiques : perte de fermeté, relâchement. La peau perd de son élasticité et les traits s'affaissent. L'affaissement des tissus sous cutanés (graisses et muscles) entraîne un excès de peau et une ptôse. Cet affaissement est caractérisé par un glissement des pommettes et des joues, entraînant la paupière inférieure. Associé à la contrainte mécanique de surface, des rides se forment puis s'accentuent et deviennent plus profondes. De plus, au cours de la journée, en fonction des mouvements hydriques dus à la gravité, certaines rides s'amplifient et deviennent plus marquées.

Parmi les rides coexistant sur le visage, on peut ainsi distinguer les rides embryonnaires qui sont issues de petits points au départ invisibles à l'oeil nu, qui se rejoignent avec le temps pour former une ride ; les rides profondes et marquées, résultat du creusement de certains sillons au cours du temps ; et les rides réversibles, qui proviennent de la diminution au cours de la journée de l'épaisseur de la peau et de l'augmentation de son élasticité.

Un certain nombre de composés a déjà été identifié en tant qu'actifs anti-rides et mis en oeuvre dans des compositions cosmétiques à des fins de lutter contre les signes cutanés du vieillissement, et en particulier diminuer et/ou effacer les rides de la peau.

Par exemple, le rétinol présente une efficacité certaine en tant qu'actif anti-ride, notamment de part ses propriétés anti-différentiantes. Toutefois, le rétinol est un actif qui n'agit qu'à long terme. Par ailleurs, pour assurer la tolérance en cosmétique, le taux de rétinol introduit dans les compositions cosmétiques est restreint, ce qui limite son efficacité.

Pour suppléer en partie à ces insuffisances, des associations du rétinol à d'autres composants ont déjà été proposées, et notamment à d'autres actifs reconnus pour leur activité anti-rides, telle que l'adénosine connue pour son activité de type « retinol-like» vis-à-vis de l'enzyme TGK.

Ainsi, il est proposé dans la demande de brevet JP 10007541 une lotion destinée à lutter contre les effets sur la peau liés à un stress oxydatif, et notamment les rides, comprenant notamment, de la vitamine A (rétinol) et de l'adénosine.

On connaît également de la demande EP 1 847 547 l'utilisation de dérivés d'adénosine dans des compositions cosmétiques à des fins de lutter contre les rides, notamment les rides d'expression, et/ou décontracter la peau et/ou détendre les traits de la peau. Il est envisagé l'ajout de divers composés additionnels tels que le rétinol et ses dérivés.

Toutefois, l'efficacité de ce type d'associations n'est pas toujours immédiate. En d'autres termes, elle ne va répondre généralement au traitement des rides qu'à moyen ou long terme. L'effet visible de ces compositions ne se produit ainsi qu'au bout d'un certain temps d'application, pouvant aller de plusieurs jours à plusieurs semaines.

La présente invention vise précisément à suppléer à cette insuffisance, et permet en particulier d'accélérer la manifestation d'un effet visible sur la diminution et/ou l'effacement des rides de la peau.

Plus précisément, l'invention concerne, selon un de ses premiers aspects, une composition cosmétique ou dermatologique comprenant dans un milieu physiologiquement acceptable, une association contenant au moins un rétinoïde, un composé non-phosphaté à base d'adénosine et un polymère semi-cristallin à chaîne(s) latérale(s) cristallisable(s).

Il est connu du document WO 2007/141142, la mise en oeuvre d'un polymère semi-cristallin, en particulier un polymère semi-cristallin à chaîne(s) cristallisable(s), en association avec un polymère d'acide 2-acrylamido 2-méthylpropane sulfonique comprenant au moins un motif hydrophobe, dans des émulsions H/E. Une telle association autorise la formulation d'émulsions H/E stables et avantageusement dépourvues de tensioactifs, conciliant les avantages procurés par la présence de substances grasses telles que des cires, bénéfiques pour nourrir la peau, avec une texture agréable, une application aisée et une sensation de fraîcheur à l'application. Les compositions cosmétiques incorporant une telle association peuvent, par exemple, être des produits de soin pour traiter les rides et les signes du vieillissement cutané, hydrater la peau ou traiter les peaux sensible ou sèches. Toutefois, il n'est nullement envisagé dans ce document qu'un polymère semi-cristallin à chaîne(s) latérale(s) cristallisable(s) puisse être utilisé en lui-même, en combinaison avec au moins un rétinoïde et au moins un composé non-phosphaté à base d'adénosine, à des fins de renforcer l'efficacité anti-rides de compositions cosmétiques ou dermatologiques.

Les inventeurs ont constaté qu'une association d'actifs selon l'invention permettait de lutter efficacement contre les signes du vieillissement cutané liés à une peau ridée ou relâchée, et en particulier d'estomper efficacement les rides, avec un effet immédiat visible, et un effet renforcé à court, moyen et long terme.

Contre toute attente, les inventeurs ont ainsi constaté que la présence d'un polymère semi-cristallin à chaîne(s) latérale(s) cristallisable(s) permet d'accéder à une structuration de la composition particulièrement avantageuse au regard de l'effet comblement et resurfaçant des rides, notamment recherché selon l'invention, ainsi que des effets soft-focus intrinsèques (flou, matité, etc...) qui parallèlement, permettent de troubler la perception visuelle des rides. Il en ressort un effet camouflage quasi immédiat des rides qui en se manifestant conjointement avec l'effet anti-rides de l'association rétinoïde et adénosine, permet de renforcer significativement l'efficacité anti-rides des compositions selon l'invention au regard de compositions déjà existantes.

Une telle association d'actifs permet en particulier d'agir efficacement sur la texture de la peau, et dé procurer notamment une peau plus lisse, plus homogène, plus ferme, tonique et élastique.

Ainsi, selon un autre de ses aspects, la présente invention concerne l'utilisation, notamment cosmétique, d'une association telle que décrite précédemment pour prévenir et/ou traiter les signes du vieillissement cutané.

Les signes du vieillissement cutané sont notamment une perte de fermeté, de densité, d'élasticité, de tonicité de la peau, un amincissement de l'épiderme, un relâchement, un affaissement de tissus cutanés et/ou sous-cutanés, de type ptôse par exemple, les ridules et les rides.

Les compositions selon l'invention sont plus particulièrement avantageuses pour prévenir et/ou traiter les signes cutanés du vieillissement, notamment tels que définis ci-dessus et plus particulièrement choisis parmi la peau ridée et/ou la peau relâchée.

Ainsi, selon encore un autre de ses aspects, la présente invention concerne l'utilisation d'une association telle que décrite précédemment pour prévenir et/ou traiter la peau ridée et/ou la peau relâchée.

Une telle association est notamment particulièrement efficace pour diminuer et/ou effacer les rides embryonnaires et/ou profondes et/ou réversibles, telles que définies précédemment.

Les rides concernées peuvent par exemple être celles disposées radialement autour de la bouche et/ou des yeux, en particulier les rides de la patte d'oie, les rides sous l'oeil et/ou situées au niveau du front, en particulier la ride dite du lion, située au niveau de la gabelle, dans l'espace inter-sourcillier, et/ou disposées horizontalement sur le front, les rides du sillon nasogénien, et la ptôse du bas du visage.

Selon un autre de ses aspects, l'invention vise un procédé de traitement non thérapeutique ou cosmétique de la peau, notamment humaine, destiné à prévenir et/ou traiter les signes cutanés du vieillissement, notamment les rides, comprenant l'application sur la peau d'une association et/ou d'une composition telles que définies précédemment.

Avantageusement, la composition selon l'invention est destinée à être appliquée sur les zones du visage et/ou du front marqués par des rides.

Le procédé de l'invention est plus particulièrement destiné à être mis en oeuvre pour des personnes présentant des signes de vieillissement cutané, et notamment des personnes à peau ridée et/ou relâchée.

Selon un mode de réalisation de l'invention, l'association considérée selon l'invention peut être mise en oeuvre avec un actif anti-âge, et notamment anti-rides, annexe.

Au sens de la présente invention, on entend par « actif anti-rides » un composé d'origine naturelle ou synthétique produisant un effet biologique, tel que l'augmentation de la synthèse et/ou de l'activité de certaines enzymes, lorsqu'il est mis en contact avec une zone de peau ridée, cet effet ayant pour conséquence de réduire l'apparence des rides et/ou ridules.

Les compositions pouvant être mises en oeuvre dans le cadre de la présente invention peuvent être des compositions cosmétiques, pharmaceutiques et/ou dermatologiques et plus particulièrement sont des compositions cosmétiques.

Au sens de la présente invention, une composition cosmétique désigne une composition apte à produire un effet au niveau de la peau sur le plan esthétique et du confort, ou encore à visée beauté, par exemple en vue de la protéger, de la maintenir en bon état, d'en modifier l'aspect, et notamment de l'embellir.

Selon une variante de l'invention, il s'agit plus particulièrement d'une composition destinée à une application topique.

### RETINOIDES

Le rétinoïde selon l'invention peut être le rétinol (vitamine A), le rétinal (vitamine A aldéhyde), l'acide rétinoïque (acide de la vitamine A), ou un ester de rétinol et d'un acide en C₂-C₂₀, tel que le propionate, l'acétate, le linoléate, ou le palmitate de rétinol (palmitate de rétinyle):

Parmi les rétinoïdes, on peut plus particulièrement citer le rétinol, le rétinal, l'acide rétinoïque, en particulier l'acide all-trans rétinoïque et l'acide 13-cis rétinoïque, les dérivés de rétinol tels que l'acétate, le propionate ou le palmitate de rétinol et les rétinoïdes décrits dans les demandes de brevets suivantes : FR 2 570 377, EP 0 199 636, EP 0 325 540 et EP 0 402 072.

Selon une forme d'exécution préférée de l'invention, le rétinoïde est le rétinol ou un pro-rétinol.

Par le terme « rétinol », on entend tous les isomères du rétinol, à savoir le rétinol all-trans, le rétinol 13-cis, le rétinol 11-cis, le rétinol 9-cis et le 3,4-didéhydrorétinol.

A titre représentatif de composés pro-rétinol, on peut notamment citer le palmitate de rétinyle (palmitate de rétinol).

Conviennent tout particulièrement ceux commercialisés par la société BASF.

Selon un mode de réalisation, le rétinoïde peut être présent dans une composition de l'invention à raison de 0,005 à 5 % en poids, de préférence de 0,01 à 2 % en poids, et notamment de 0,05 à 0,5 % en poids par rapport au poids total de la composition.

### COMPOSES NON-PHOSPHATES À BASE D'ADENOSINE

Une composition de l'invention comprend au moins un composé non-phosphaté à base d'adénosine.

Par cette expression, on entend aussi bien l'adénosine elle-même que ses dérivés non phosphatés connus.

A titres d'exemples de dérivés non phosphatés d'adénosine, on peut citer : l'adénosine ; la 2'-deoxyadénosine ; la 2', 3'-isopropoylidene adénosine; la toyocamycine ; la 1-méthyladénosine ; la N-6-methyladénosine ; l'adénosine N-oxyde; le 6-méthylmercaptopurine riboside ; et le 6-chloropurine riboside.

D'autres dérivés d'adénosine comprennent les agonistes des récepteurs à l'adénosine dont la phénylisopropyl-adénosine ("PIA"), la 1-méthylisoguanosine, la N⁶-Cyclohexyladénosine (CHA), la N⁶-cyclopentyladénosine (CPA), la 2-chloro-N6-cyclopentyladénosine, la 2-chloroadénosine, la N⁶-phényladénosine, la 2-phénylaminoadénosine, la MECA, la N⁶-phénéthyladénosine, la 2-p-(2-carboxy-éthyl) phénéthyl-amino-5'-N-éthylcarboxamido-adénosine (CGS-21680), la N-éthylcarboxamido-adénosine (NECA), la 5' (N-cyclopropyl)-carboxamidoadénosine, la DPMA (PD 129,944) et le metrifudil.

D'autres dérivés d'adénosine comprennent les composés qui augmentent la concentration intracellulaire d'adénosine tels que l'érythro-9-(2-hydroxy-3-nonyl) adénine ("EHNA") et l'iodotubercidine.

Les dérivés d'adénosine décrits dans la demande EP 1 847 547 sont également incorporés par référence.

D'autres dérivés encore d'adénosine comprennent ses sels et alkyl esters.

L'adénosine est préférée pour une utilisation dans la présente invention. Elle est notamment disponible dans le commerce sous forme de poudre auprès de la société PHARMA WALDHOF.

Dans les compositions conformes à l'invention, le composé à base d'adénosine représente de préférence de 0,0001 % à 5 % en poids, plus préférentiellement de 0,001 à 1 % en poids et, mieux de 0,01 % à 0,5 % en poids, par rapport au poids total de la composition.

### POLYMERES SEMI-CRISTALLINS A CHAINE(S) LATERALE(S) CRISTALLISABLE(S)

Une composition selon l'invention comprend au moins un polymère semi-cristallin à chaîne(s) latérale(s) cristallisable(s), de préférence choisi parmi les homopolymères de (méth)acrylates d'alkyle en C₁₀-C₃₀.

Comme précisé ci-dessus, la présence d'un tel polymère est notamment avantageuse au regard de l'effet texturation qu'il procure à la composition. Celle-ci permet, en effet, de procurer un effet lissage immédiat au niveau de la peau sur laquelle elle est appliquée.

Cette texturation est plus particulièrement réalisée au niveau de la phase grasse de la composition, dans laquelle est formulé ledit polymère.

Par « polymères », on entend au sens de l'invention des composés comportant au moins 2 motifs de répétition, de préférence au moins 3 motifs de répétition, et plus spécialement au moins 10 motifs de répétition.

Par « polymère semi-cristallin », on entend au sens de l'invention, des polymères comportant au moins une chaîne pendante cristallisable présentant une température de changement de phase réversible du premier ordre, en particulier de fusion (transition solide-liquide).

Par « chaîne cristallisable », on entend une chaîne comportant au moins 10 atomes de carbone, et qui, si elle était seule, passerait de l'état amorphe à l'état cristallin, de façon réversible, selon qu'on est au-dessus ou en dessous de la température de fusion. Une chaîne au sens de l'invention est un groupement d'atomes, pendant ou latéral par rapport au squelette du polymère.

Un polymère semi-cristallin convenant à l'invention peut présenter en particulier une température de fusion supérieure à la température de la peau.

Selon un mode particulier de réalisation, un polymère semi-cristallin à chaîne(s) latérale(s) cristallisable(s) convenant à l'invention peut présenter une température de fusion inférieure ou égale à 80 °C, et en particulier comprise dans une plage allant de 30 °C à 70 °C, en particulier de 40 °C à 65 °C, plus particulièrement de 42 °C à 60 °C, encore de 44 °C à 56 °C, voire de 44 °C à 54 °C, et plus particulièrement inférieure à 50 °C.

Cette température ou point de fusion (Pf) peut être mesurée par toute méthode connue et en particulier avec un calorimètre à balayage différentiel (D.S.C).

Selon un mode particulier de réalisation de l'invention, le polymère peut être choisi parmi les homopolymères résultant de la polymérisation d'au moins un monomère à chaîne latérale cristallisable choisis parmi les (méth)acrylates d'alkyle saturés en C₁₀ à C₃₀, qui peut être représenté par la formule suivante : 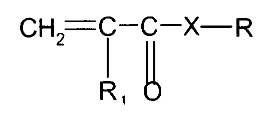 dans laquelle R₁ est H ou CH₃, R représente un groupe alkyle en C₁₀ à C₃₀, et X représente O.

Selon un mode plus particulier de réalisation de l'invention, le polymère est issu de la polymérisation de monomères à chaîne cristallisable choisi parmi les (méth)acrylates d'alkyle saturés en C₁₀ à C₃₀, et plus particulièrement les homopolymères résultant de la polymérisation d'un monomère à chaîne cristallisable choisi parmi les acrylates d'alkyle en C₁₄-C₂₄ et les méthacrylates d'alkyle en C₁₄-C₂₄.

A titre d'exemple particulier de polymères semi-cristallins utilisables dans une composition selon l'invention, on peut citer les produits Intelimer TM de la société Landec décrits dans la brochure « Intelimer TM polymers », Landec IP22. Ces polymères sont sous forme solide à température ambiante. Ils portent des chaînes latérales cristallisables et correspondent à des homopolymères d'acrylates ou méthacrylates d'alkyle en C₁₄-C₂₄ saturé.

On peut également citer comme polymère semi-cristallin convenant aux compositions selon la présente invention, l'homopolymère d'acrylate de stéaryle tel que celui commercialisé sous la dénomination Intelimer^{®} IPA 13-1, de la société LANDEC ou de la société AIR PRODUCT and CHEMICALS, ou l'homopolymère d'acrylate de béhényle (Intelimer IPA-13.6) (nom INCI : Poly C10-30 alkyl acrylate).

La texturation de la composition peut être avantageusement ajustée en fonction de la nature des polymères et leurs concentrations respectives.

En particulier, la quantité en polymère(s) semi-cristallin est ajustée de manière à procurer le lissage attendu à l'application de la composition considérée sur la peau.

Selon un mode de réalisation avantageux de l'invention, une composition de l'invention peut comprendre au moins 0,6 % en poids, en particulier au moins 1 % en poids de polymère semi-cristallin à chaîne(s) latérale(s) cristallisable(s) comme décrits ci-dessus par rapport au poids total de la composition, mieux de 1 à 5 %, encore mieux de 1 à 3 % en poids de polymère semi-cristallin à chaîne(s) latérale(s) cristallisable(s) par rapport au poids total de la composition.

L'homme de l'art saura ajuster la présence de ces derniers de sorte à ne pas affecter substantiellement les propriétés requises des compositions de l'invention.

Selon un mode de réalisation particulier de l'invention, une composition selon l'invention comprend du rétinol ou palmitate de rétinol en association avec de l'adénosine et un homopolymère de (méth)acrylate en C₁₀-C₃₀, en particulier le polyacrylate de stéaryle.

Selon une variante de réalisation, les compositions selon l'invention peuvent associer audit polymère semi-cristallin à chaîne(s) latérale(s) cristallisable(s), au moins un polymère annexe, notamment utile en terme de propriétés de texturation.

### POLYMERE ADDITIONNEL

Une composition de l'invention peut également comprendre, outre le polymère semi-cristallin à chaîne(s) latérale(s) cristallisable(s) décrit précédemment, un polymère additionnel choisi parmi les polymères acryliques, les polysaccharides modifiés hydrophobes et les esters d'acide gras et de polyols.

De préférence le polymère additionnel est hydrophile.

### • Polymères acryliques

Ces polymères acryliques peuvent être hydrophiles ou non.

### a- Polymères acryliques hydrophiles

Par « polymères acryliques hydrophiles » selon l'invention, on entend notamment des polymères acryliques non hydrophobes et non amphiphiles.

Lesdits polymères acryliques hydrophiles selon l'invention sont soit des polymères acryliques d'acide polyacrylamidomethyl propane sulfonique (AMPS^{®}) soit des polymères acryliques acides.

La présence de ce polymère acrylique hydrophile permet notamment d'obtenir une composition présentant de bonnes propriétés de stabilité.

Parmi les polymères acryliques hydrophiles, on peut notamment citer les polymères suivants.

### 1) Polymères acryliques comportant au moins un monomère à groupement sulfonique

Selon un premier mode de réalisation, le polymère acrylique hydrophile utilisé selon l'invention comporte au moins un monomère à groupement sulfonique.

Les polymères comportant au moins un monomère à groupement sulfonique, utilisés dans la composition de l'invention, sont avantageusement hydrosolubles ou hydrodispersibles ou gonflables dans l'eau. Les polymères utilisés conformément à l'invention sont des homopolymères susceptibles d'être obtenus à partir d'au moins un monomère à insaturation éthylénique et à groupement sulfonique, pouvant être sous forme libre ou partiellement ou totalement neutralisée.

De façon préférentielle, les polymères conformes à l'invention peuvent être neutralisés partiellement ou totalement par une base minérale (soude, potasse, ammoniaque) ou une base organique telle que la mono-, di- ou tri-éthanolamine, un aminométhylpropanediol, la N-méthyl-glucamine, les acides aminés basiques comme l'arginine et la lysine, et les mélanges de ces composés. Ils sont généralement neutralisés. On entend dans la présente invention par « neutralisés », des polymères totalement ou pratiquement totalement neutralisés, c'est-à-dire neutralisés à au moins 90 %.

Les polymères utilisés dans la composition de l'invention ont généralement un poids moléculaire moyen en nombre allant de 1000 à 20 000 000 g/mole, de préférence allant de 20 000 à 5 000 000 et plus préférentiellement encore de 100 000 à 1 500 000 g/mole.

Ces polymères selon l'invention peuvent être réticulés ou non réticulés.

Les monomères à groupement sulfonique du polymère utilisé dans la composition de l'invention sont choisis notamment parmi l'acide vinylsulfonique, l'acide styrènesulfonique, les acides (méth)acrylamido-(C₁-C₂₂)alkylsulfoniques, les acides N-(C₁-C₂₂)alkyl-(méth)acrylamido-(C₁-C₂₂)-alkylsulfoniques comme l'acide undécyl-acrylamido-méthane-sulfonique, ainsi que leurs formes partiellement ou totalement neutralisées, et leurs mélanges.

Selon un mode préféré de réalisation de l'invention, les monomères à groupement sulfonique sont choisis parmi les acides (méth)acrylamido(C₁-C₂₂)alkylsulfoniques tels que par exemple l'acide acrylamido-méthane-sulfonique, l'acide acrylamido-éthane-sulfonique, l'acide acrylamido-propane-sulfonique, l'acide 2-acrylamido-2-méthylpropane-sulfonique, l'acide 2-méthacrylamido-2-méthylpropane-sulfonique, l'acide 2-acrylamido-n-butane-sulfonique, l'acide 2-acrylamido-2,4,4-triméthylpentane-sulfonique, l'acide 2-méthacrylamido-dodécyl-sulfonique, l'acide 2-acrylamido-2,6-diméthyl-3-heptane-sulfonique, ainsi que leurs formes partiellement ou totalement neutralisées, et leurs mélanges.

Plus particulièrement, on utilise l'acide 2-acrylamido-2-méthylpropane-sulfonique (AMPS^{®}) ainsi que ses formes partiellement ou totalement neutralisées.

Lorsque les polymères sont réticulés, les agents de réticulation peuvent être choisis parmi les composés à polyinsaturation oléfinique couramment utilisés pour la réticulation des polymères obtenus par polymérisation radicalaire.

On peut citer par exemple comme agents de réticulation, le divinylbenzène, l'éther diallylique, le dipropylèneglycol-diallyléther, les polyglycol-diallyléthers, le triéthylèneglycol-divinyléther, l'hydroquinone-diallyl-éther, le di(méth)acrylate de d'éthylèneglycol ou de tétraéthyléneglycol, le triméthylol propane triacrylate, le méthylène-bis-acrylamide, le méthylène-bis-méthacrylamide, la triallylamine, le triallylcyanurate, le diallylmaléate, la tétraallyléthylènediamine, le tétra-allyloxy-éthane, le triméthylolpropane-diallyléther, le (méth)acrylate d'allyle, les éthers allyliques d'alcools de la série des sucres, ou d'autres allyl- ou vinyl- éthers d'alcools polyfonctionnels, ainsi que les esters allyliques des dérivés de l'acide phosphorique et/ou vinylphosphonique, ou les mélanges de ces composés.

Selon un mode préféré de réalisation de l'invention, l'agent de réticulation est choisi parmi le méthylène-bis-acrylamide, le méthacrylate d'allyle ou le triméthylol propane triacrylate (TMPTA). Le taux de réticulation va en général de 0,01 à 10% en mole et plus particulièrement de 0,2 à 2 % en mole par rapport au polymère.

L'homopolymère de monomères à groupement sulfonique peut être réticulé avec un ou plusieurs agents de réticulation.

Ces homopolymères sont généralement réticulés et neutralisés, et ils peuvent être obtenus selon le procédé de préparation comprenant les étapes suivantes :
(a) on disperse ou on dissout le monomère tel que l'acide 2-acrylamido 2-méthylpropane sulfonique sous forme libre dans une solution de tertio-butanol ou d'eau et de tertio-butanol ;
(b) on neutralise la solution ou la dispersion de monomère obtenue en (a) par une ou plusieurs bases minérales ou organiques, de préférence l'ammoniaque NH₃, dans une quantité permettant d'obtenir un taux de neutralisation des fonctions acides sulfoniques du polymère allant de 90 à 100% ;
(c) on ajoute à la solution ou dispersion obtenue en (b), le ou les monomères réticulants ;
(d) on effectue une polymérisation radicalaire classique en la présence d'amorceurs de radicaux libres à une température allant de 10 à 150°C ; le polymère précipitant dans la solution ou la dispersion à base de tertio-butanol.

Les homopolymères d'AMPS^{®} préférés sont en général caractérisés par le fait qu'ils comprennent, distribués de façon aléatoire :
a) de 90 à 99,9 % en poids de motifs de formule générale (II) suivante : 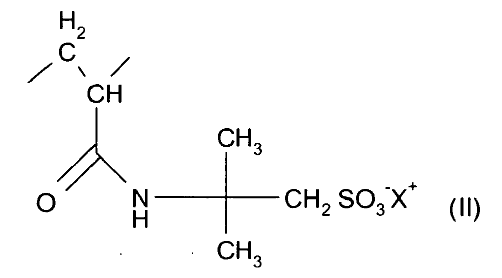 dans laquelle X⁺ désigne un proton, un cation de métal alcalin, un cation alcalino-terreux ou l'ion ammonium, au plus 10% mol des cations X⁺ pouvant être des protons H⁺ ;
b) de 0,01 à 10% en poids de motifs réticulants provenant d'au moins un monomère ayant au moins deux double-liaison oléfiniques ; les proportions en poids étant définis par rapport au poids total du polymère.

Les homopolymères selon l'invention plus particulièrement préférés comprennent de 98 à 99,5 % en poids de motifs de formule (II) et de 0,2 à 2 % en poids de motifs réticulants.

Comme polymères de ce type, on peut citer notamment l'homopolymère réticulé et neutralisé d'acide 2-acrylamido 2-méthylpropane sulfonique, commercialisé par la société Clariant sous la dénomination commerciale « Hostacerin^{®} AMPS » (nom CTFA : ammonium polyacryldimethyltauramide).

### 2) Copolymères acrylamide/AMPS

Selon un autre mode de réalisation, le polymère acrylique hydrophile est un copolymère anionique réticulé constitué de motifs dérivant de la réaction entre (i) l'acrylamide (monomère 1), (ii) l'acide 2-acrylamido 2-méthyl propane sulfonique (monomère 2, ci-après dénommé par commodité AMPS^{®}) et (iii) au moins un composé à polyinsaturation oléfinique (monomère 3), constituant ici l'agent de réticulation.

Les copolymères anioniques réticulés utilisés dans le cadre de la présente invention sont des produits déjà connus en soi et leur préparation a été décrite notamment dans la demande de brevet EP-A-0 503 853, dont le contenu est par conséquent totalement inclus à titre de référence dans la présente description.

Les copolymères ci-dessus peuvent ainsi être obtenus classiquement selon la technique dite de polymérisation en émulsion à partir des trois différents comonomères rentrant dans leur constitution.

Les monomères à polyinsaturation oléfinique utilisés comme agents de réticulation pour la préparation des copolymères conformes à l'invention sont de préférence choisis dans le groupe constitué par le méthylène bis-acrylamide, l'allyle-sucrose et le pentaérythritol. Encore plus préférentiellement, on fait appel au méthylène bis-acrylamide.

De préférence, ledit composé à polyinsaturation oléfinique est présent dans le copolymère à une concentration comprise entre 0,06 et 1 millimole par mole de l'ensemble des motifs monomères.

Le rapport, exprimé en moles %, entre l'acrylamide et l'AMPS^{®} est préférentiellement compris entre 85/15 et 15/85, avantageusement entre 70/30 et 30/70, encore plus préférentiellement entre 65/35 et 35/65, et plus particulièrement encore entre 60/40 et 40/60. En outre, l'AMPS^{®} est généralement au moins partiellement neutralisé sous la forme d'un sel, par exemple par de la soude, par de la potasse, ou par une amine à faible poids moléculaire telle que la trièthanolamine, ou leurs mélanges.

Un copolymère réticulé particulièrement préféré dans le cadre de la mise en oeuvre de la présente invention correspond à celui tel que préparé à l'exemple 1 de la demande de brevet EP-A- 0 503 853 précitée, et qui se présente alors sous la forme d'une émulsion inverse eau-dans-huile. Plus précisément, ce copolymère est constitué de 60 % en moles d'acrylamide et de 40 % en moles de sel de sodium d'AMPS^{®}, et il est réticulé par du méthylène bis-acrylamide à raison de 0,22 millimole par mole du mélange total de monomères. L'émulsion inverse finale eau-dans-huile contient, quant à elle, et de préférence, environ 40 % en poids de copolymère réticulé tel que défini ci-avant et de l'ordre de 4 % en poids d'un alcool gras éthoxylé ayant un HLB d'environ 12,5.

On utilise plus particulièrement selon l'invention comme copolymère réticulé les produits vendus sous les noms Sepigel 305 (nom C.T.F.A. : polyacrylamide/C13-14 isoparaffin/Laureth 7) ou Simulgel 600 (nom C.T.F.A. : acrylamide / sodium acryloyldimethyltaurate copolymer / isohexadecane / polysorbate 80) vendus par la société Seppic.

### 3) Autres polymères acryliques hydrophiles

Comme autres polymères acryliques hydrophiles utilisables selon l'invention, on peut également citer :
- les homo- ou copolymères d'acides acrylique ou leurs sels, tels que les produits vendus sous les dénominations Carbopol 934, 940, 954, 981, 980 par la société NOVEON, le Synthalen L^{®} de la société 3V, le polyméthacrylate de sodium vendu sous la dénomination DARVAN N°7^{®} par la société VANDERBILT ;
- les polymères d'acrylate de glycéryle, et en particulier les copolymères d'acrylate de glycéryle et d'acide acrylique, tels que les produits vendus sous les dénominations. « LUBRAJEL^{®} MS », « LUBRAJEL^{®} CG », « LUBRAJEL^{®} DV », « LUBRAJEL^{®} NP », « LUBRAJEL^{®} OIl », », « LUBRAJEL^{®} Oil BG », « LUBRAJEL^{®} PF », « LUBRAJEL^{®} TW », « LUBRAJEL^{®} WA » par la société Guardian Laboratories. On utilise de préférence le « LUBRAJEL^{®} MS ».
- les copolymères de sel d'acide acrylique/ alcool vinylique, tel que le produit vendu sous la dénomination Hydragen FN^{®} de Cognis,
et leurs mélanges.

### b- Polymère acryliques hydrophobes

De tels polymères peuvent dérivés des AMPS^{®} décrits précédemment. Ces polymères comportent à la fois une partie hydrophile et une partie hydrophobe comportant au moins une chaîne grasse. Il s'agit donc de polymères amphiphiles.

Une chaîne grasse d'un tel polymère peut comporter de 7 à 30 atomes de carbone, et en particulier de 8 à 22 atomes de carbone.

Un copolymère d'AMPS^{®} hydrophobé conforme à l'invention peut avoir un poids moléculaire en poids allant de 50 000 à 10 000 000, en particulier de 100 000 à 8 000 000 et plus particulièrement de 100 000 à 7 000 000.

Un copolymère d'AMPS^{®} hydrophobé selon l'invention peut être réticulé ou non réticulé.

Parmi les agents de réticulation susceptibles de convenir, on peut mentionner, de manière non limitative, le méthylène-bis-acrylamide, le méthacrylate d'allyle ou le triméthylol propane triacrylate (TMPTA).

Le taux de réticulation peut varier de 0,01 à 10 % en moles et en particulier de 0,2 à 2 % en moles par rapport au polymère.

Un polymère d'AMPS^{®} amphiphile convenant à l'invention peut, par exemple, être choisi parmi des polymères amphiphiles statistiques d'AMPS^{®} modifiés par réaction avec une N-monoalkylamine ou une di-N-alkylamine en C₆-C₂₂ tels que ceux décrits dans la demande de brevet WO 00/31154.

Ces polymères peuvent également contenir d'autres monomères hydrophiles à insaturation éthylénique choisis par exemple parmi l'acide acrylique, l'acide méthacrylique ou leurs dérivés alkyl-substitués ou leurs esters obtenus avec des mono ou polyalkylèneglycols, l'acrylamide, le méthacrylamide, la vinylpyrrolidone, l'acide itaconique ou l'acide maléique ou leurs mélanges.

Un polymère de l'invention peut être choisi parmi des polymères amphiphiles d'AMPS^{®} et d'au moins un monomère à insaturation éthylénique comportant au moins une partie hydrophobe ayant de 7 à 30 atomes de carbone, et en particulier de 8 à 22 atomes de carbone, et plus particulièrement de 12 à 20 atomes de carbone.

La partie hydrophobe peut être un radical alkyle linéaire, saturé ou insaturé (par exemple n-octyle, n-décyle, n-hexadécyle, n-dodécyle, oléyle), ramifié (par exemple isostéarique) ou cyclique (par exemple cyclododécane ou adamantane).

Un polymère convenant à l'invention peut contenir, en outre, un ou plusieurs co-monomères hydrophobes à insaturation éthylènique, comprenant par exemple :
- un radical fluoré ou alkylfluoré en C₇-C₁₈ (par exemple le groupement de formule -(CH₂)₂-(CF₂)₉-CF₃),
- un radical cholestéryle ou un radical dérivé de cholestérol (par exemple l'hexanoate de cholestéryle),
- un groupe polycyclique aromatique comme le naphtalène ou le pyrène,
- un radical siliconé ou alkylsiliconé ou encore alkylfluorosiliconé.

De tels copolymères sont décrits par exemple dans la demande de brevet EP-A-0 750 899, le brevet US-A-5,089,578 et dans les publications de Yotaro Morishima : "Self-assembling amphiphilic polyelectrolytes and their nanostructures - Chinese Journal of Polymer Science Vol. 18, N(40), (2000): 323-336" ; "Micelle formation of random copolymers of sodium 2-(acrylamido)-2- methylpropanesulfonate and a non-ionic surfactant macromonomer in water as studied by fluorescence and dynamic light scattering - Macromolecules 2000, Vol. 33 N(10) : 3694-3704" ; "Solution properties of micelle networks formed by non-ionic moieties covalently bound to an polyelectrolyte : salt effects on rheological behavior- Langmuir, 2000,Vol.16 N(12): 5324-5332" ; "Stimuli responsive amphiphilic copolymers of sodium 2-(acrylamido)-2-methylpropanesulfonate and associative macromonomers - Polym. Preprint, Div. Polym. Chem. 1999, 40(2): 220-221 ". Ils sont également décrits dans les demandes de brevet (CLARIANT) : EP 1 069 142, WO 02/44224, WO 02/44225, WO 02/44227, WO 02/44229, WO 02/44230, WO 02/44231, WO 02/44267, WO 02/44268, WO 02/44269, WO 02/44270, WO 02/44271, WO 02/43677, WO 02/43686, WO 02/43687, WO 02/43688, WO 02/43689.

Un monomère hydrophobe à insaturation éthylénique convenant à l'invention peut être choisi parmi les acrylates ou les acrylamides de formule (1) suivante : 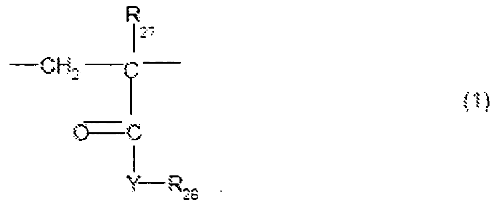 dans laquelle :
- R₂₇ désigne un atome d'hydrogène, un radical alkyle linéaire ou ramifié en C₁-C₆ (de préférence méthyle),
- Y désigne O ou NH,
- R₂₈ désigne un radical hydrophobe comportant une chaîne grasse ayant de 7 à 30 atomes de carbone, en particulier de 8 à 22, et plus particulièrement de 12 à 20 atomes de carbone.

Le radical hydrophobe R₂₈ peut être en particulier choisi parmi les radicaux alkyles linéaires en C₇-C₂₂, saturés ou insaturés (par exemple n-octyle, n-décyle, n-hexadécyle, n-dodécyle, oléyle), ramifiés (par exemple isostéarique) ou cycliques (par exemple cyclododécane ou adamantane) ; les radicaux alkylperfluorés en C₇-C₁₈ (par exemple le groupement de formule -(CH₂)₂-(CF₂)₉-CF₃) ; le radical cholestéryle ou un ester de cholestérol comme l'hexanoate de cholestéryle ; les groupes polycycliques aromatiques comme le naphtalène ou le pyrène.

Parmi ces radicaux, les radicaux alkyles linéaires et ramifiés seront plus particulièrement mis en oeuvre.

Selon un mode de réalisation de l'invention, le radical hydrophobe R₂₈ peut comporter en plus au moins un motif oxyde d'alkylène, et en particulier une chaîne polyoxyalkylénée.

Une chaîne polyoxyalkylénée peut être constituée de motifs oxyde d'éthylène et/ou de motifs oxyde de propylène, et plus particulièrement peut être constituée uniquement de motifs oxyde d'éthylène.

Le nombre de moles de motifs oxyalkylénés peut varier en général de 1 à 30 moles et plus particulièrement de 2 à 25 moles, et encore plus particulièrement de 3 à 20 moles.

Parmi les polymères amphiphiles d'AMPS^{®} convenant à l'invention, on peut citer :
- les copolymères réticulés ou non réticulés, neutralisés ou non, comportant de 15 à 60 % en poids de motifs AMPS^{®} et de 40 à 85 % en poids de motifs (C₈-C₁₆)alkyl(méth)acrylamide ou de motifs (C₈-C₁₆)alkyl(méth)acrylate par rapport au polymère, tels que ceux décrits dans la demande EP-A-750 899 ;
- les terpolymères comportant de 10 à 90 % en mole de motifs acrylamide, de 0,1 à 10 % en mole de motifs AMPS^{®} et de 5 à 80 % en mole de motifs n-(C₆-C₁₈)alkylacrylamide, par rapport au polymère, tels que ceux décrits dans le brevet US-A-5,089,578.

Comme polymères amphiphiles convenant à l'invention, on peut également citer les copolymères d'AMPS^{®} totalement neutralisé et de méthacrylate de n-dodécyle, de n-hexadécyle et/ou de noctadécyle, ainsi que les copolymères d'AMPS^{®} et de n-dodécylméthacrylamide, non réticulés et réticulés.

On peut également citer les copolymères amphiphiles réticulés ou non réticulés comprenant, voire constitués:
(a) des motifs acide 2-acrylamido 2-méthylpropane sulfonique (AMPS^{®}) de formule (2) suivante : 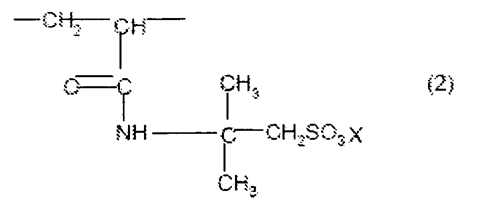 dans laquelle X peut être un proton, un cation de métal alcalin, un cation alcalino-terreux ou l'ion ammonium ;
(b) et des motifs de formule (3) suivante : 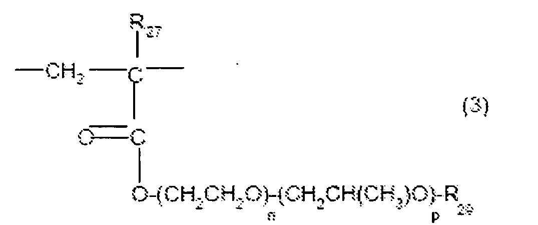 dans laquelle :
   - n et p, indépendamment l'un de l'autre, désignent un nombre de moles et varient de 0 à 30, en particulier de 1 à 25 et plus particulièrement de 3 à 20, sous réserve que n + p soit inférieur ou égal à 30, en particulier inférieur à 25 et plus particulièrement inférieur à 20 ;
   - R₂₇ a la même signification indiquée ci-dessus dans la formule (1) précédente, et
   - R₂₉ désigne un alkyle linéaire ou ramifié comportant m atomes de carbone, m allant de 7 à 22, de préférence de 12 à 20.

Dans la formule (2), le cation X peut désigner plus particulièrement le sodium ou l'ammonium.

Parmi les monomères de formule (3) on peut citer :
- les esters d'acide (méth)acrylique et d'alcool gras en C₁₀-C₁₈ polyoxethyléné à 8 OE comme le produit GENAPOL C-080 vendu par la Société CLARIANT,
- les esters d'acide (méth)acrylique et d'oxoalcool gras en C₁₁ polyoxyéthyléné à 8 OE comme le produit GENAPOL UD-080 vendu par la Société CLARIANT,
- les esters d'acide (méth)acrylique et d'alcool gras polyoxyéthyléné en C₁₂-C₁₄ à 7 OE comme le produit GENAPOL LA-070 vendu par la Société CLARIANT,
- les esters d'acide (méth)acrylique et d'alcool gras polyoxyéthyléné en C₁₂-C₁₄ à 11 OE comme le produit GENAPOL LA-110 vendu par la Société CLARIANT,
- les esters d'acide (méth)acrylique et d'alcool gras polyoxyéthyléné en C₁₆-C₁₈ à 8 OE comme le produit GENAPOL T-080 vendu par la Société CLARIANT,
- les esters d'acide (méth)acrylique et d'alcool gras polyoxyéthyléné en C₁₆-C₁₈ à 15 OE comme le produit GENAPOL T-150 vendu par la Société CLARIANT,
- les esters d'acide (méth)acrylique et d'alcool gras polyoxyéthyléné en C₁₆-C₁₈ à 11 OE comme le produit GENAPOL T-110 vendu par la Société CLARIANT,
- les esters d'acide (méth)acrylique et d'alcool gras polyoxyéthyléné en C₁₆-C₁₈ à 20 OE comme le produit GENAPOL T-200 vendu par la Société CLARIANT,
- les esters d'acide (méth)acrylique et d'alcool gras polyoxyéthyléné en C₁₆-C₁₈ à 25 OE comme le produit GENAPOL T-250 vendu par la Société CLARIANT,
- les esters d'acide (méth)acrylique et d'alcool gras polyoxyéthyléné en C₁₈-C₂₂ à 25 OE et/ ou d'isoalcool gras polyoxyéthyléné en C₁₆-C₁₈ à 25 OE.

On peut plus particulièrement citer :
i. les polymères non réticulés pour lesquels p= 0, n = 7 ou 25, R₂₇ désigne méthyle et R₂₉ représente un mélange d'alkyle en C₁₂-C₁₄ ou en C₁₆-C₁₈, et
ii. les polymères réticulés pour lesquels p = 0, n = 8 ou 25, R₂₇ désigne méthyle et R₂₉ représente un mélange d'alkyle en C₁₆-C₁₈.

Ces polymères sont décrits et synthétisés dans le document EP 1 069 142.

Ces polymères amphiphiles particuliers peuvent être obtenus selon les procédés classiques de polymérisation radicalaire en présence d'un ou plusieurs initiateurs, tels que par exemple, l'azobisisobutyronitrile (AIBN), l'azobisdiméthylvaléronitrile, le chlorhydrate de 2,2-azobis-[2-amidinopropane], l'ABAH (2,2-azo-bis-[2-amidinopropane] hydrochloride), les peroxydes organiques tels que le peroxyde de dilauryle, le peroxyde de benzoyle, l'hydroperoxyde de tert-butyle, etc..., des composés peroxydés minéraux tels que le persulfate de potassium ou d'ammonium, ou H₂O₂ éventuellement en présence de réducteurs.

Ces polymères amphiphiles peuvent être obtenus par polymérisation radicalaire en milieu tert-butanol dans lequel ils précipitent.

En utilisant la polymérisation par précipitation dans le tert-butanol, il est possible d'obtenir une distribution de la taille des particules du polymère particulièrement favorable pour ses utilisations.

La réaction peut être conduite à une température comprise entre 0 et 150 °C, en particulier entre 10 et 100 °C, soit à pression atmosphérique, soit sous pression réduite.

La réaction peut aussi être réalisée sous atmosphère inerte, et de préférence sous azote.

Un polymère conforme à l'invention peut être neutralisé partiellement ou totalement par une base minérale ou organique telle que celles citées ci dessus.

La concentration molaire en % des motifs de formule (2) et des motifs de formule (3) dans un polymère amphiphile selon l'invention peut varier en fonction de l'application cosmétique souhaitée, de la nature de l'émulsion (huile-dans-eau ou eau-dans-huile) et des propriétés rhéologiques de la formulation recherchée.

Elle peut varier entre 0,1 et 99,9 % en moles.

La proportion molaire en motifs de formule (3) dans un polymère amphiphile selon l'invention peut varier de préférence de 0,1 à 50 %, plus particulièrement de 1 à 25 % et encore plus particulièrement de 3 à 10 %.

La proportion molaire en motifs de formule (3) dans un polymère amphiphile selon l'invention peut varier de préférence de 50,1 à 99,9 %, plus particulièrement de 60 à 95 % et encore plus particulièrement de 65 à 90 %.

La distribution des monomères dans les polymères de l'invention peut être, par exemple, alternée, bloc (y compris multibloc) ou quelconque.

A titre indicatif et sans que cela soit limitatif, on peut citer les références commerciales suivantes : Aristoflex^{®} HMS, et Aristoflex^{®} HMB commercialisées par Clariant, ces deux références se rapportant à des polymères réticulés.

L'Aristoflex ® HMS porte le nom de copolymère AMPS^{®}/méthacrylate de cetearyl éthoxyle(25 EO) 80/20, réticulé par triméthylolpropane triacrylate (TMPTA) ou encore de Ammonium Acryloyldimethyltaurate/Steareth-25 methacrylate crosspolymer en nom INCI.

En nom INCI l'Aristoflex^{®} HMB est l'Ammonium Acryloyldimethyltaurate / Beheneth-25 Methacrylate Crosspolymer.

On peut également utiliser, à titre de copolymère d'AMPS^{®} hydrophobé, les copolymères d'AMPS^{®} non réticulés (Aristoflex^{®} LNC ou SNC) qui sont aussi efficaces en terme de stabilisation des émulsions. En revanche, les textures alors obtenues sont moins originales, notamment du point de vue de l'effet de transformation en eau.

En nom INCI, l'Aristoflex^{®} LNC est l'Ammonium Acryloyldimethyltaurate/Laureth-7 Methacrylate Copolymer.

En nom INCI, l'Aristoflex^{e} SNC est l'Ammonium Acryloyldimethyltaurate/Steareth-8 Methacrylate Copolymer.

Parmi les polymères acryliques susceptibles d'être associés à une association conforme à l'invention peuvent également être cités les homo-ou copolymères acryliques réticulés neutralisés.

### c- Homo-ou copolymères acryliques réticulés neutralisés

Le rôle principal de ce polymère présent dans la composition de l'invention réside dans la gélification de la phase aqueuse.

Tous les homo- ou copolymères acryliques réticulés conviennent à la présente invention pour autant qu'ils soient mis en oeuvre sous une forme au moins partiellement neutralisée.

Au titre de ces polymères acryliques réticulés déjà neutralisés avant leur mise en oeuvre, ou non, on cite par exemple :
- le Cosmedia SP^{®} ou polyacrylate de sodium réticulé contenant 90 % de matière sèche et 10% d'eau, le Cosmedia SPL^{®} ou polyacrylate de sodium en émulsion inverse contenant environ 60 % de matière active sèche, une huile (polydecene hydrogené) et un tensio-actif (PPG-5 Laureth-5), tous deux vendus par la société Cognis,
- les carbopols,
- les polyacrylates de sodium réticulés partiellement neutralisés se trouvant sous forme d'une émulsion inverse comprenant au moins une huile polaire, par exemple celui vendu sous la dénomination Luvigel^{®} EM vendu par la société BASF, et
- leurs mélanges.

Un polymère d'acide acrylique réticulé conforme à la présente invention, non préalablement neutralisé, peut être neutralisé par tout moyen approprié et notamment par ajout de soude. On obtient ainsi des polyacrylates de sodium. Les polyacrylates de potassium conviennent également à la présente invention.

En réalité, la neutralisation peut être effectuée préalablement à la mise en oeuvre dans la composition de l'invention si le polymère en question est vendu sous une forme non neutralisée. En revanche, pour certains d'entre eux, la neutralisation est inhérente à la matière première. C'est le cas notamment du Luvigel^{®} EM et des produits nommés Cosmédia^{®} SP et SPL qui est sont déjà partiellement neutralisés.

L'étape de neutralisation, par exemple par les contre-ions sodium ou potassium, est nécessaire pour conférer aux polymères acides réticulés leurs propriétés de gélification et donc de stabilisation de la composition. Lesdits polymères acryliques réticulés sont convertis en polymères acrylates correspondants lors de cette étape de neutralisation. Les monomères acryliques du polymère acrylique réticulé selon l'invention peuvent être neutralisés à raison de 5 à 80 %.

Selon un mode de réalisation particulier de l'invention, le polymère acrylique réticulé conforme à l'invention peut comprendre des monomères ioniques. Au titre de monomères ioniques, on peut mettre en oeuvre des acrylamide, methacrylamide, vinylpyrrolidone, vinylimidazole, vinylcarpolactam et des esters hydroxyalkyl d'acides carboxyliques tels que des hydroxyéthylacrylates. Comme monomère ionique on cite en particulier les acides carboxyliques en C₃-C₅ insaturés. Toutefois, on privilégie dans le cadre de la présente invention les polymères acryliques réticulés comprenant plus de 90 % de monomères d'acide acrylique, voire ne comprenant aucun monomère non-ionique.

Selon un mode de réalisation particulier, l'homo- ou copolymère d'acide acrylique réticulé peut se trouver sous forme d'une émulsion eau-dans-huile, dite émulsion inverse. Cette émulsion inverse peut par exemple être obtenue par polymérisation en émulsion inverse.

Selon un mode de réalisation particulier de l'invention, le polymère gélifiant mis en oeuvre est un polyacrylate de sodium réticulé partiellement neutralisé se trouvant sous forme d'une émulsion inverse comprenant au moins une huile polaire. Parmi les huiles on peut citer les esters d'acides gras. Des exemples de ces esters d'acides gras sont des esters ispropyliques d'acides gras, tels que le palmitate d'isopropyle ou le myristate d'isopropyle, ou des polyglycérides d'acides gras, en particulier de mélanges d'acides gras comprenant au moins 50 % d'acides caprique et/ou caprylique. De telles émulsions eau-dans-huile sont décrites dans le document US 6,197,283, qui est incorporé dans la présente demande par référence.

Selon ce mode de réalisation, la phase huileuse peut être constituée d'un ou plusieurs esters d'acides gras, d'un ou plusieurs polyglycérides d'acide gras à base d'un mélange de polyglycérides, qui contient des diglycérides et des triglycérides, avec des mélanges d'acides gras, qui contiennent de l'acide caprylique et/ou de l'acide caprique, de préférence à raison d'au moins 50 % en poids par rapport au poids total d'acides gras.

Selon un mode de réalisation de l'invention, la teneur en huile de l'émulsion inverse est comprise entre 15 et 70 % en poids, en particulier entre 20 et 35 % en poids par rapport au poids total de l'émulsion inverse.

A ce titre on cite notamment le Luvigel^{®} EM, dont la phase huileuse comprend 26 % de phase huile constituée de triglycérides en C₈-C₁₀, à savoir dont les acides gras sont un mélange d'acide caprique et caprylique.

Par ailleurs, l'émulsion eau-dans-huile peut contenir de 0,25 à 7 % en poids, de préférence 0,5 à 5% en poids, d'un agent tensioactif.

Le polymère acrylique réticulé au moins partiellement neutralisé peut être présent dans l'émulsion inverse dans une teneur variant de 20 % à 70 % en poids, en particulier de 20 à 65 % en poids, par exemple de 20 à 62 % en poids par rapport au poids total de l'émulsion inverse.

Notamment, selon un mode de réalisation, le polymère acrylique réticulé peut être présent dans l'émulsion inverse dans une teneur variant de 20 à 30 % en poids par rapport au poids total de l'émulsion inverse. Selon encore un autre mode de réalisation, le polymère acrylique réticulé peut être présent dans l'émulsion inverse dans une teneur variant de 50 à 62 % en poids par rapport au poids total de la composition.

Les polymères conformes à l'invention peuvent être constitués
a) de 35 à 100 % en poids de monomères ioniques, les monomères ioniques étant neutralisés à 5-80 %,
b) de 0 à 65 % en poids de monomères non ioniques,
c) de 0,3 à 1 % molaire, par rapport à a) et b), d'au moins un monomère au moins bifonctionnel.

Dans la formulation eau-dans-huile d'un tel polymère, la phase huileuse peut alors être constituée d'un ou plusieurs esters d'acide gras tels que décrits précédemment.

La réticulation de l'acide acrylique peut être obtenue selon tout méthode connue de l'homme de l'art, notamment selon la description du document US 6,197,283 ou selon la description du document US 6,444,785 qui mentionne les agents de réticulation utilisables.

Parmi eux, on cite les composés comportant une insaturation solubles dans l'eau ou dans l'huile. De tels agents de réticulation sont notamment le méthylenbisacrylamide, le divinylpyrrolidone, l'alkyl(méth)acrylate, la triallylamine, les diacrylates d'éthylenglycol (jusqu'à 50 OE), les esters (méth)acryliques avec des alcools di- ou polyhydriques tels que le triacrylate de triméthylolpropane ou le tétraacrylate de pentaerythritol.

Selon un mode de réalisation, l'agent de réticulation est soluble dans l'eau.

Selon un autre mode de réalisation, l'agent de réticulation est la triallylamine.

La préparation d'émulsions E/H comprenant un polymère conforme à la présente invention peut être effectuée selon l'enseignement du document US6,444,785, incorporé ici par référence. Ce procédé a pour objectif d'abaisser la teneur en monomères restants par post-traitement avec un système d'initiateur redox. Selon ce procédé, on effectue le post-traitement de l'émulsion E/H par addition d'un système d'initiateur redox qui comporte pour l'essentiel
a) 0,001 à 5 % en poids, par rapport à la quantité totale de monomères utilisée pour la préparation du polymère
   a1) d'un agent d'oxydation R¹OOH,
   dans lequel R¹ désigne l'hydrogène, un groupe alkyle en C₁ à C₈ ou un groupe aryle en C₆ à C₁₂, et/ ou
   a2) d'un composé libérant du peroxyde d'hydrogène en milieu aqueux, et
b) 0,005 à 5 % en poids, par rapport à la quantité totale de monomères utilisée pour la préparation du polymère,
   b1) d'un composé d'α-hydroxycarbonyle 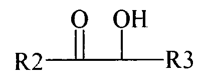 dans lequel les groupements ont, indépendamment l'une de l'autre, la signification suivante:
      R2 hydrogène, un groupe alkyle en C₁-C₁₂, qui contient éventuellement des groupes fonctionnels et/ou peut comporter des insaturations oléfiniques,
      R3 hydrogène, OH, un groupe alkyle en C₁-C₁₂, qui contient éventuellement des groupes fonctionnels et/ou peut comporter des insaturations oléfiniques,
      et tandis que R2 et R3 peuvent former une structure cyclique, qui peut contenir un hétéroatome et/ou des groupes fonctionnels, et/ou peut comporter des insaturations oléfiniques,
      et/ou
   b2) d'un composé libérant un tel composé d'α-hydroxy-carbonyle en milieu aqueux,et
c) des quantités catalytiques d'un ion métallique plurivalent pouvant se présenter sous plusieurs états de valence.

### • Polysaccharides modifiés hydrophobes

Par « polysaccharide modifié hydrophobe » selon l'invention, on entend notamment un polysaccharide modifié par des chaînes hydrophobes, en particulier modifié par greffage de chaînes hydrophobes sur le squelette hydrophile dudit polysaccharide.

Par « polysaccharide » selon l'invention, on entend notamment les inulines les celluloses et leurs dérivés (méthylcelluloses, hydroxyalkylcelluloses, hydroxyethylcelluloses, hydroxypropylcelluloses, éthylhydroxyéthylcelluloses, carboxyméthyl-celluloses), les amidons, les agars, et leurs mélanges.

De préférence, on utilisera les inulines, les celluloses et leurs dérivés, et leurs mélanges. Une composition de l'invention peut ainsi comprendre un polysaccharide modifié hydrophobe choisi parmi les inulines, les celluloses et leurs dérivés, les amidons, les agars et leurs mélanges.

### Inulines modifiées hydrophobes

Selon un premier mode de réalisation, le polysaccharide utilisé dans la présente invention est choisi parmi les fructanes, en particulier les inulines.

Les fructanes ou fructosanes sont des oligosaccharides ou des polysaccharides comprenant un enchaînement d'unités anhydrofructose éventuellement associé à un plusieurs résidus saccharidiques différents du fructose. Les fructanes peuvent être linéaires ou ramifiés. Les fructanes peuvent être des produits obtenus directement à partir d'une source végétale ou microbienne ou bien des produits dont la longueur de chaîne a été modifiée (augmentée ou réduite) par fractionnement, synthèse ou hydrolyse en particulier enzymatique. Les fructanes ont généralement un degré de polymérisation de 2 à environ 1000 et de préférence de 2 à environ 60.

On distingue 3 groupes de fructanes. Le premier groupe correspond à des produits dont les unités fructose sont pour la plupart liées par des liaisons β-2-1.Ce sont des fructanes essentiellement linéaires tes que les inulines.

Le second groupe correspond également à des fructoses linéaires mais les unités fructose sont essentiellement liées par des liaisons β-2-6. Ces produits sont des levanes.

Le troisième groupe correspond à des frucanes mixtes, c'est à dire ayant des enchainements β-2-6 et β-2-1. Ce sont des fructanes essentiellement ramifiés tes que les graminanes.

Les fructanes utilisés dans les compositions selon l'invention sont les inulines. L'inuline peut être obtenue par exemple à partir de chicorée, de dahlia ou de topinambours. De préférence, l'inuline utilisée dans la composition selon l'invention est obtenue par exemple à partir de chicorée.

Les polysaccharides, en particulier les inulines, utilisés dans les compositions selon l'invention sont modifiés hydrophobes. En particulier, elles sont obtenues par greffage de chaînes hydrophobes sur le squelette hydrophyle du fructane.

Les chaînes hydrophobes susceptibles d'être greffées sur la chaîne principale du fructane peuvent notamment être des chaînes hydrocarbonées linéaires ou ramifiées, saturées ou insaturées, ayant de 1 à 50 atomes de carbone, telles que les groupements alkyle, arylalkyle, alkylaryle, alcoylène ; des groupements divalents cycloaliphatiques ou des chaînes organopolysiloxanes. Ces chaînes hydrocarbonées ou organopolysiloxanes peuvent notamment comprendre une ou plusieurs fonctions ester, amide, uréthane, carbamate, thiocarbamate, urée, thio-urée, et/ou sulfonamide tels que notamment méthylènedicyclohexyl et isophorone ; ou des groupements divalents aromatiques tels que phénylène.

Une composition de l'invention peut ainsi comprendre comme polysaccharide modifié hydrophobe est une inuline modifiée par des chaînes hydrophobes linéaires ou ramifiées, saturées ou insaturées, ayant de 1 à 50 atomes de carbone, telles que les groupements alkyle, arylalkyle, alkylaryle, alcoylène ; des groupements divalents cycloaliphatiques ou des chaînes organopolysiloxanes comprenant une ou plusieurs fonctions ester, amide, uréthane, carbamate, thiocarbamate, urée, thio-urée, et/ou sulfonamide tels que notamment méthylènedicyclohexyl et isophorone ; ou des groupements divalents aromatiques tels que phénylène.

En particulier, l'inuline est obtenue à partir de chicorée.

En particulier, le polysaccharide, notamment de l'inuline, présente un degré de polymérisation de 2 à environ 1000 et de préférence de 2 à environ 60, et un degré de substitution inférieur à 2 sur la base d'une unité fructose.

Selon un mode préféré de réalisation, les chaînes hydrophobes présentent au moins un groupement alkyle carbamate de formule R-NH-CO- dans laquelle R est groupement alkyle ayant de 1 à 22 atomes de carbone.

Selon un mode plus préféré de réalisation, les chaines hydrophobes sont des groupements lauryle carbamate.

En particulier, à titre illustratif et non limitatif des inulines modifiées hydrophobes pouvant être utilisées dans les compositions selon l'invention, on peut citer la stéaroyl inuline telle que celles vendues sous les dénominations Lifidrem INST par la société Engelhard et Rheopearl INS par la société Ciba ; la palmitoyl inuline ; l'undécylénoyl inuline telle que celles vendues sous les dénominations Lifidrem INUK et Lifidrem INUM par la société Engelhard ; et l'inuline lauryl carbamate tel que celui vendu sous la dénomination INUTEC SP1 1 par la société ORAFTI.

En particulier, le polysaccharide modifié hydrophobe est une inuline greffée lauryl carbamate, notamment issue de la réaction d'isocyanate de lauryle sur une inuline, en particulier issue de la chicorée. A titre d'exemple de ces composés, on peut notamment citer le produit vendu sous la dénomination INUTEC SP1 par la société ORAFTI.

### Dérivés de cellulose modifiés hydrophobes

Selon un autre mode de réalisation de l'invention, le polysaccharide modifié hydrophobe est une hydroxyalkyle cellulose (C₁-C₃) modifiée par des chaînes hydrophobes, en particulier des groupements hydrophobe(s) comprenant de 8 à 30 atomes de carbone.

Les dérivés de cellulose modifiés hydrophobes selon l'invention sont substitués, par une ou plusieurs chaîne(s) hydrocarbonée(s) en C₈-C₃₀ linéaires, ramifiées ou cycliques, saturées ou insaturées, aliphatiques ou aromatiques.

Selon un mode de réalisation, le ou les substituants hydrophobes utilisés sont des groupes alkyle, arylalkyle ou alkylaryle en C₈-C₃₀, de préférence en C₁₀-C₂₂.

De préférence, le ou les substituants hydrophobes selon la présente invention sont des chaînes alkyles saturées en C₁₀-C₂₂, de préférence en C₁₆-C₂₀, tels que des groupements cétyl (C₁₆), stearyl (C₁₈) et béhényl (C₂₀).

Selon un mode de réalisation préféré, le ou les substituants hydrophobes selon la présente invention sont des groupements cétyl.

Ces dérivés de cellulose à substituant(s) hydrophobe(s) selon l'invention, présentent une viscosité de préférence comprise entre 100 et 100 000 mPa.s, et de préférence entre 200 et 20 000 mPa.s, mesurée à 25 °C dans une solution à 1% en poids de polymère dans l'eau, cette viscosité étant déterminée de manière conventionnelle à l'aide d'un viscosimètre de type Brookfield LVT à 6 tours par minute avec le mobile n° 3.

Parmi les dérivés de cellulose à substituant(s) hydrophobe(s) utilisables dans les compositions de l'invention, on peut citer, de préférence, les cétyl hydroxyéthylcelluloses commercialisées sous les dénominations Natrosol Plus Grade 330 CS et Polysurf 67 CS (INCI : Cetyl Hydroxyethylcellulose) par la société Aqualon/Hercules.

### • Esters d'acide gras et de polyols

Par « esters d'acide gras et de polyols » selon l'invention, on entend des esters d'acide gras (ou polymères d'acide gras) et de polyol dans lesquels l'acide gras comprend une chaîne alkyle en C₆-C₂₂, de préférence en C₁₆-C₂₀, et le polyol est choisi parmi le glycérol, un polyglycérol, le sorbitan, et leurs mélanges. L'acide gras peut également être sous une forme polymérique, comme c'est le cas de l'acide polyhydroxystéarique (polymère de l'acide 12-hydroxystéarique).

Selon un mode particulier, l'ester d'acide gras et de polyol est un ester d'acide gras en C₁₆-C₂₀ et de glycérol et/ou de sorbitan, et leurs mélanges.

Comme exemples d'acides gras à chaîne linéaire ou ramifiée en C₁₆-C₂₀, on peut citer l'acide stéarique, l'acide isostéarique, l'acide laurique, l'acide myristique, l'acide palmitique. Comme exemple de polymère d'acide gras en C₁₆-C₂₀, on peut citer l'acide poly 12-hydroxystéarique.

De préférence on utilisera l'acide stéarique, isostéarique, l'acide poly 12-hydroxystéarique et leurs mélanges.

Par polyglycérols, on entend des composés de formule : 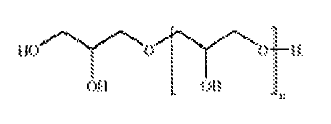 dans laquelle le degré de condensation n va de 1 à 11, de préférence de 2 à 6 et encore plus préférentiellement de 3 à 6.

Selon un mode particulier, l'ester d'acide gras et de polyol contient 2 à 10 moles (unités) de polyols, de préférence 2 à 4 moles de polyols, en particulier 2 à 4 unités de glycérol ou un mélange de polyglycérols (glycérol, di-, tri-, tetra-, penta-, oligoglycerols).

Encore plus préférentiellement, l'ester d'acide gras et de polyol contient 4 moles (ou unités) de poltol, en particulier 4 moles (ou unités) de glycérol.

Selon un mode préféré, ledit ester d'acide gras et de polyol est en outre un ester d'acide gras, de diacide carboxylique ayant de 2 à 16 atomes de carbone, de préférence de 8 à 14 atomes de carbone, tels que l'acide azélaïque, l'acide sébacique, l'acide dodecanedioique, et de préférence l'acide sébacique (C₁₀, et de polyol.

A titre d'exemples d'esters d'acide gras et de polyol utilisables dans la composition de l'invention, on peut citer les esters d'acide isostéarique et de polyols et leurs mélanges, en particulier les esters d'acide isostéarique et de glycérol et/ou de sorbitan, tels que par exemple l'isostéarate polyglycérolé (4 moles) (nom INCI : Polyglyceryl-4 Isostearate) vendu sous la dénomination Isolan G134^{®} par la société Goldschmidt, le diisostéarate polyglycérolé (3 moles) vendu sous la dénomination Lameform TGI^{®} par la société Cognis ; le distéarate polyglycérolé (2 moles) vendu sous la dénomination Emalex PGSA^{®} par la société Nihon emulsion ; le monoisostearate polyglycérolé (10 moles) vendu sous la dénomination Nikkol decaglyn 1-IS par la société Nihon Surfactant (Nom INCI : Polyglyceryl-10 isostearate) ; le polyglycéryl-4 diisostéarate polyhydroxystéarate sébacate vendu sous la dénomination ISOLAN GPS par Goldschmidt ; le mélange d"isostéarate de sorbitan et d'isostéarate de glycérol, tel que le produit commercialisé sous la dénomination Arlacel 986 par la société ICI, le mélange d'isostéarate de sorbitan et d'isostéarate de polyglycérol (3 moles) commercialisé sous la dénomination Arlacel 1690 par la société Unigema, et leurs mélanges.

Comme esters d'acide gras et de polyglycérol préférés selon l'invention, on peut citer notamment : l'isostéarate polyglycérolé (4 moles) (nom INCI : Polyglyceryl-4 Isostearate) vendu sous la dénomination Isolan G134^{®} par Goldschmidt, le polyglycéryl-4 diisostéarate polyhydroxystéarate sébacate vendu sous la dénomination Isolan GPS^{®} par Goldschmidt, le mélange d'isostéarate de sorbitan et d'isostéarate de polyglycérol (3 moles) commercialisé sous la dénomination Arlacel 1690^{®} par la société Unigema, et leurs mélanges.

Selon un mode préféré de l'invention, l'ester d'acide gras et de polyol selon l'invention est un ester d'acide poly12-hydroxystéarique et d'acides dicarboxyliques obtenus par estérification d'un mélange de polyglycérol avec (i) un acide polyhydroxystéarique, avec de 1 à 10, de préférence de 2 à 8, encore plus préférentiellement de 2 à 5 unités de polyglycérol (de préférence 4 unités); (ii) des acides dicarboxyliques aliphatiques, linéaires ou ramifiés ayant de 2 à 16 atomes de carbone, de préférence de 4 à 14 atomes de carbone (de préférence l'acide sébacique); et (iii) des acides gras saturés ou insaturés, linéaires ou ramifiés ayant de 6 à 22 atomes de carbone, de préférence de 16 à 20 atomes de carbone (de préférence l'acide isostéarique).

Avantageusement, le degré d'estérification du mélange de polyglycérol est entre 20 et 40 %, de préférence entre 40 et 70 %.

Des tels esters d'acide poly12-hydroxystéarique et de polyglycérol sont décrits dans la demande US 2005/0031580.

Selon un mode préféré, l'ester d'acide gras et de polyol est un ester d'acide poly12-hydroxystéarique et d'acides dicarboxyliques obtenus par estérification d'un mélange de polyglycérol avec (i) un acide polyhydroxystéarique, avec de 2 à 5 unités de polyglycérol; (ii) des acides dicarboxyliques aliphatiques, linéaires ou ramifiés ayant 4 à 14 atomes de carbone et (iii) des acides gras saturés ou insaturés, linéaires ou ramifiés ayant de 16 à 20 atomes de carbone.

De préférence, l'ester d'acide gras et de polyol est un ester d'acide polyhydroxystéarique et d'acides dicarboxyliques obtenus par estérification d'un mélange de polyglycérol avec (i) un acide polyhydroxystéarique, avec de 2 à 5 unités de polyglycérol (de préférence 4 unités); (ii) des acides dicarboxyliques aliphatiques, linéaires ou ramifiés ayant 4 à 14 atomes de carbone carbone (de préférence l'acide sébacique) et (iii) des acides gras saturés ou insaturés, linéaires ou ramifiés ayant de 16 à 20 atomes de carbone (de préférence l'acide isostéarique).

Comme exemple préféré d'ester d'acide polyhydroxystéarique et de polyglycérol, on peut citer le polyglycéryl-4 diisostéarate polyhydroxystéarate sébacate de formule 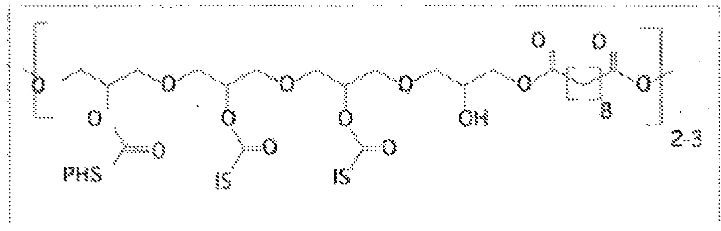 où PHS désigne l'acide polyhydroxystéarique et IS désigne l'acide isostéarique.

Un tel composé est préparé selon la demande US 2005/0031580 et commercialisé sous la dénomination ISOLAN GPS^{®} par la société GOLDSCHMIDT (DEGUSSA).

Selon un mode de réalisation particulier de l'invention, une composition selon l'invention est dépourvue de polymère d'acide 2-acrylamido 2-méthylpropane sulfonique comprenant au moins un motif hydrophobe.

### Actifs cosmétiques complémentaires

Selon un mode de réalisation avantageux, l'association selon l'invention peut être associée à un ou plusieurs actifs cosmétiques complémentaires.

Ces actifs pourront être choisis parmi les agents anti-rides autre que l'adénosine et le rétinol, les filtres UV, les vitamines en particulier B3, B8, B12 et B9, les agents hydratants, les agents desquamants, les actifs anti-âges, les agents améliorant la fonction barrière, les agents dépigmentants, les agents antioxydants, les agents dermodécontractants ou agents dermorelaxants, les agents anti-glycation, les agents stimulant la synthèse des macromolécules dermiques et/ou épidermiques et/ou empêchant leur dégradation, les agents stimulant la prolifération des fibroblastes ou des kératinocytes et/ou la différenciation des kératinocytes, les agents favorisant la maturation de l'enveloppe cornée, les inhibiteurs de NO-synthases, les antagonistes des récepteurs périphériques des benzodiazépines (PBR), les agents augmentant l'activité de la glande sébacée, les agents stimulant le métabolisme énergétique des cellules et les agents apaisants.

La composition selon l'invention pourra aussi contenir des actifs pour le soin ou le traitement des peaux grasses.

Selon une variante particulière de l'invention, l'association selon l'invention est mise en oeuvre conjointement avec au moins un actif choisi parmi les actifs anti-rides autres que l'adénosine, le rétinol et leurs dérivés, les filtres UV, les agents desquamants, les agents antioxydants, les actifs stimulant la synthèse des macromoléculaires dermiques et/ou épidermiques, les agents dermodécontractants et leurs mélanges.

Ces actifs complémentaires pourront être présents dans la composition en une teneur allant de 0,001 à 20 % en poids, de préférence de 0,01 à 10 % en poids, et plus préférentiellement de 0,01 à 5 % en poids par rapport au poids total de la composition.

A titre illustratif des actifs anti-âges, peuvent être notamment cités le Pro-Xylane ou des extraits de graines de *vigna aconitifolia* comme ceux commercialisés par la société Cognis sous les références Vitoptine LS9529 et Vit-A-Like LS9737.

### Autres agents anti-rides

Des exemples d'actifs anti-rides additionnels utilisables selon l'invention sont: l'acide ascorbique et ses dérivés, tels que l'ascorbyl phosphate de magnésium et le glucoside d'ascorbyle; le tocophérol et ses dérivés, tels que l'acétate de tocophéryle; l'acide nicotinique et ses précurseurs, tels que la nicotinamide; l'ubiquinone; le glutathion et ses précurseurs, tels que l'acide L-2-oxothiazolidine-4-carboxylique; les composés C-glycoside et leurs dérivés, tels que décrits notamment ci-après ; les extraits de plantes et notamment les extraits de criste marine et de feuille d'olivier, ainsi que les protéines végétales et leurs hydrolysats, tels que les hydrolysats de protéines de riz ou de soja ; les extraits d'algues et en particulier de laminaires ; les extraits bactériens ; les sapogénines, telles que la diosgénine et les extraits de Dioscorées, en particulier de Wild Yam, en contenant ; les α-hydroxyacides; les β-hydroxyacides, tels que l'acide salicylique et l'acide n-octanoyl-5-salicylique; les oligopeptides et pseudodipeptides et leurs dérivés acylés, en particulier l'acide {2-[acetyl-(3-trifluoromethyl-phenyl)-amino]-3-methyl-butyrylamino} acétique et les lipopeptides commercialisés par la société SEDERMA sous les dénominations commerciales Matrixyl 500 et Matrixyl 3000 ; le lycopène; les sels de manganèse et de magnésium, en particulier les gluconates; et leurs mélanges.

Une composition de l'invention peut mettre en oeuvre notamment du sodium hyaluronate à titre d'agent anti-âge.

L'actif anti-rides additionnel peut représenter au moins 0,05 %, de préférence au moins 0,5 % et plus préférentiellement au moins 1% en poids par rapport au poids total de la composition.

### Agents hydratants

Comme agents humectants ou hydratants, on peut citer notamment l'urée et ses dérivés notamment l'Hydrovance^{®} commercialisée par National Starch, des monosaccharides comme le mannose, l'acide hyaluronique, les AHA, les BHA, des homopolymères d'acide acrylique comme le Lipidure-HM^{®} de NOF corporation, le beta-glucan et en particulier le sodium carboxymethyl beta-glucane de Mibelle-AG-Biochemistry ; un polyoxybutylène polyoxyéthylène polyoxypropylène glycérol comme le WILBRIDE S-753L^{®} de NOF corporation, une huile de rosier muscat commercialisée par Nestlé; des sphères de collagène et de chondroitine sulfate d'origine marine (Ateocollagen) commercialisées par la société Engelhard Lyon sous la dénomination sphères de comblement marines; des sphères d'acide hyaluronique telles que celles commercialisées par la société Engelhard Lyon.

### Filtres UV

A titre d'illustration des filtres UV et de façon non limitative, on peut citer les familles suivantes :
les anthranilates, en particulier l'anthranilate de menthyle ; les benzophénones, en particulier la benzophénone-1, la benzophénonc-3, la benzophénone-5, la benzophénone-6, la benzophénone-8, la benzophénone-9, la benzophénone-12, et préférentiellement la Benzophénone-2 (Oxybenzone), ou la Benzophénone-4 (Uvinul MS40^{®} disponible chez B.A.S.F.) ; les benzylidènes-camphres, en particulier le 3-benzylidène-camphre, l'acide benzylidènecampho-sulfonique, le benzalkoniumméthosulfate de Camphre, le polyacrylamidométhylbenzylidène camphre, l'acide téréphthalylidène di-camphre sulfonique , et préférentiellement le 4-méthylbenzylidène camphre (Eusolex 6300^{®} disponible chez Merck) ; les benzimidazoles, en particulier le benzimidazilate (Neo Heliopan AP^{®} disponible chez Haarmann et Reimer), ou l'acide phénylbenzimidazole sulfonique (Eusolex 232^{®} disponible chez Merck) ; les benzotriazoles, en particulier le drométrizole trisiloxane, ou le méthylène bis-benzotriazolyltétraméthylbutylphénol (Tinosorb M^{®} disponible chez Ciba) ; les cinnamates, en particulier le cinoxate, le DEA méthoxycinnamate, le méthylcinnamate de diisopropyle, le glycéryl éthylhexanoate de diméthoxycinnamate, le méthoxycinnamate d'isopropyle, le cinnamate d'isoamyle, et préférentiellement l'éthocrylène (Uvinul N35^{®} disponible chez B.A.S.F.), l'octylméthoxycinnamate (Parsol MCX^{®} disponible chez Hoffmann La Roche), ou l'octocrytène (Uvinul 539^{®} disponible chez B.A.S.F.) ; les dibenzoylméthanes, en particulier le butyl méthoxydibenzoylméthane (Parsol 1789^{®}); les imidazolines, en particulier l'éthylhexyl diméthoxybenzylidène dioxoimidazoline; les PABA, en particulier l'éthyl Dihydroxypropyl PABA, l'éthylhexyldiméthyl PABA, le glycéryl PABA, le PABA, le PEG-25 PABA, et préférentiellement la diéthylhexylbutamido-triazone (Uvasorb HEB^{®} disponible chez 3V Sigma), l'éthylhexyltriazone (Uvinul T150^{®} disponible chez B.A.S.F.), ou l'éthyl PABA (benzocaïne); le Mexoryl^{®}; les salicylates, en particulier le salicyclate de dipropylèneglycol, le salicylate d'éthylhexyle, l'homosalate, ou le TEA salicylate ; les triazines, en particulier l'anisotriazine (Tinosorb S^{®} disponible chez Ciba) ; le drometrizole trisiloxane, l'oxyde de zinc, le dioxyde de titane, l'oxyde de zinc, de fer, de zirconium, de cerium enrobés ou non.

De préférence, on utilise les cinnamates, les salicylates et leurs mélanges.

La quantité de filtres dépend de l'utilisation finale souhaitée. Elle peut aller par exemple de 1 à 30 % en poids et mieux de 2 à 21 % en poids par rapport au poids total de la composition le contenant.

### Agents desquamants

Comme agents desquamants préférés, on pourra citer les beta-hydroxyacides, en particulier l'acide salicylique et ses dérivés autres que l'acide n-octanoyl 5-salicylique ; l'urée; les acides glycolique, citrique, lactique, tartrique, malique ou mandélique; l'acide 4-(2-hydroxyethyl)piperazine-1-propanesulfonique (HEPES) ; l'extrait de Saphora japonica; le miel ; le N-acétyl glucosamine ; le méthyl glycine diacétate de sodium, les alpha-hydroxy acides (AHA), les Beta-hydroxy acides (BHA), et leurs mélanges.

Encore plus préférentiellement on utilisera avec l'association de l'invention un agent desquamant tel que l'acide 4-(2-hydroxyethyl)piperazine-1-propanesulfonique (HEPES).

Ainsi, une composition conforme à l'invention peut comprendre un agent desquamant dans une teneur allant de 0,001 % à 10 % en poids, de préférence de 0,01 % à 5 % en poids par rapport au poids total de la composition.

### Agents dépigmentants

Comme agents dépigmentants, on peut citer les céramides, la vitamine C et ses dérivés et notamment la vit CG, CP et 3-O éthyl vitamine C, l'alpha et la béta arbutine, l'acide férulique, l'acide kojique, le résorcinol et ses dérivés, le D calcium panthéteine sulfonate, l'acide lipoique, l'acide ellagique, la vitamine B3, le phényléthyl résorcinol comme le Symwhite 377^{®} de la société Symrise, une eau de fruit de kiwi *(Actinidia chinensis)* commercialisée par Gattefosse, un extrait de racine de *Paeonia suffructicosa* tel que celui commercialisé par la société Ichimaru Pharcos sous la dénomination Botanpi Liquid B^{®}, un extrait de sucre brun *(Saccharum officinarum),* tel que l'extrait de melasse commercialisé par la société Taiyo Kagaku sous la dénomination Molasses Liquid, un mélange d'acide undecylenique et phénylalanine undecylenoyl, tel que le Sepiwhite MSH^{®} de Seppic.

### Agents antioxydants

Comme agent antioxydant préféré, on peut plus particulièrement citer le tocophérol et ses esters, en particulier l'acétate de tocophérol ; l'EDTA, l'acide ascorbique et ses dérivés, en particulier l'ascorbyl magnésium phosphate et l'ascorbyl glucoside; les chélatants, tels que le BHT, le BHA, le N,N'-bis(3,4,5-triméthoxybenzyl) éthylenediamine et ses sels, et leurs mélanges.

Plus préférentiellement, on utilisera le tocopherol et ses esters.

Ainsi, une composition conforme à l'invention peut comprendre un agent antioxydant dans une teneur allant de 0,001 % à 10 % et préférentiellement de 0,01 % à 5 % en poids par rapport au poids total de la composition.

### Agents dermorelaxants ou dermodécontractants

Comme agents dermorelaxants préférés, on peut citer tout particulièrement le gluconate de manganèse, le wild yam, la criste marine, la glycine et l'alvérine.

Une composition conforme à l'invention peut comprendre un agent dermorelaxant dans une teneur allant de 0,0001 % à 5 % et préférentiellement de 0,001% à 3 % en poids par rapport au poids total de la composition.

### Actifs stimulant la synthèse de macromolécules dermiques et/ou épidermiques et/ou empêchant leur dégradation

Comme actifs préférés stimulant la synthèse de macromolécules dermiques et/ou épidermiques et/ou empêchant leur dégradation, on peut citer : les peptides extraits de végétaux, tels que l'hydrolysat de soja commercialisé par la société BASF Beauty Care Solutions sous la dénomination commerciale Phytokine^{®} l'extrait de malt tel que commercialisé sous la dénomination Collalift^{®} par la société Engelhard Lyon ; les peptides de riz tel que le Nutripeptide^{®} de SILAB, ou encore un extrait de peptides de riz tel que la Colhibin^{®} de Pentapharm, le méthylsilanol mannuronate tel que l'Algisium C^{®} commercialisé par Exsymol ; un extrait de *vaccinium myrtillus* tel que ceux décrits dans la demande
FR-A-2 814 950 ; l'extrait de lupin commercialisé par la société SILAB sous la dénomination commerciale Structurine^{®}, et leurs mélanges.

Une composition conforme à la présente invention peut comprendre un actif stimulant la synthèse des macromolécules dans une teneur allant de 0,001 % à 10 % et préférentiellement de 0,01 % à 5 % en poids par rapport au poids total de la composition.

La composition selon l'invention pourra encore contenir de l'eau de Vichy, riche en minéraux, pour son action fortifiante, anti-radicalaire, et apaisante.

### Milieu physiologiquement acceptable

Une composition contenant une association selon l'invention peut être destinée à une application cosmétique ou pharmaceutique, particulièrement dermatologique. De préférence cette composition selon l'invention est destinée à une application cosmétique. Elle est formulée dans un milieu physiologiquement acceptable.

Le milieu physiologiquement acceptable est préférentiellement un milieu cosmétiquement ou dermatologiquement acceptable, c'est-à-dire sans odeur, couleur ou aspect désagréable, et qui ne génère pas de picotement, tiraillement ou rougeur inacceptable pour l'utilisateur.

Par milieu physiologiquement acceptable, on comprend un milieu compatible avec les matières kératiniques d'êtres humains, en l'occurrence la peau.

La composition selon l'invention peut se présenter sous toutes les formes galéniques normalement utilisées dans les domaines cosmétique et dermatologique.

Elle peut être notamment sous forme d'une solution aqueuse, hydroalcoolique, éventuellement gélifiée, d'une dispersion du type lotion éventuellement biphasée, d'une émulsion huile-dans-eau ou eau-dans-huile ou multiple, d'un gel aqueux, d'une dispersion d'huiles dans une phase aqueuse, notamment à l'aide de sphérules, ces sphérules pouvant être des particules polymériques ou mieux, des vésicules lipidiques de type ionique et/ou non-ionique, ou bien encore sous la forme d'une poudre, d'un sérum, d'une pâte ou d'un bâtonnet souple.

Ainsi, la composition peut comprendre tous les constituants usuellement employés dans l'application envisagée.

On peut notamment citer l'eau, les solvants, les huiles d'origine minérale, animale et/ou végétale notamment telles que détaillées ci-après, les cires telles que décrites ci-après, en particulier les pigments, les charges, les tensioactifs, les gélifiants, les conservateurs.

Une composition de l'invention peut avantageusement présenter un toucher ferme et compact à la prise. Elle peut être épaisse à l'application et se transformer ensuite, fondre et libérer de la fraîcheur.

Bien entendu l'homme du métier veillera à choisir ce ou ces éventuels composés complémentaires, et/ou leur quantité, de manière telle que les propriétés avantageuses des composés selon l'invention ne soient pas, ou substantiellement pas, altérées par l'adjonction envisagée.

Les compositions selon l'invention contiennent avantageusement au moins une phase grasse liquide.

Avantageusement, les compositions selon l'invention peuvent se présenter sous la forme d'émulsions.

Les compositions selon l'invention peuvent avantageusement se présenter sous forme d'une émulsion obtenue par dispersion d'une phase aqueuse dans une phase grasse (E/H) ou d'une phase grasse dans une phase aqueuse (H/E), de consistance liquide ou semi-liquide du type lait, ou de consistance molle, semi-solide ou solide du type crème ou gel, ou encore d'émulsion multiple (E/H/E ou H/E/H). Ces compositions sont préparées selon les méthodes usuelles.

Les compositions de ce type peuvent avoir la forme d'un produit de soin ou de maquillage du visage et/ou du corps, et être conditionnée par exemple sous forme de crème en pot ou de fluide en tube ou en flacon pompe.

Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant l'émulsion à obtenir (E/H ou H/E). Les émulsionnants sont généralement présents dans la composition, en une proportion pouvant aller par exemple de 0,3 à 30 % en poids, et de préférence de 0,5 à 20 % en poids par rapport au poids total de la composition.

Pour les émulsions H/E, on peut citer par exemple comme émulsionnants, les tensioactifs non ioniques, et notamment les esters de polyols et d'acide gras à chaîne saturée ou insaturée comportant par exemple de 8 à 24 atomes de carbone et mieux de 12 à 22 atomes de carbone, et leurs dérivés oxyalkylénés, c'est-à-dire comportant des unités oxyéthylénés et/ou oxypropylénés, tels les esters de glycéryle et d'acide gras en C₈-C₂₄, et leurs dérivés oxyalkylénés; les esters de polyéthylène glycol et d'acide gras en C₈-C₂₄, et leurs dérivés oxyalkylénés; les esters de sorbitol et d'acide gras en C₈-C₂₄, et leurs dérivés oxyalkylénés ; les esters de sucre (sucrose, glucose, alkylglucose) et d'acide gras en C₈-C₂₄, et leurs dérivés oxyalkylénés ; les éthers d'alcools gras ; les éthers de sucre et d'alcools gras en C₈-C₂₄, et leurs mélanges.

Comme exemples d'huiles utilisables dans la composition selon l'invention, on peut citer :
- les huiles hydrocarbonées d'origine animale, telles que le perhydrosqualène,
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité,

- les esters et les éthers de synthèse, notamment d'acides gras, comme les huiles de formules R₁COOR₂ et R₁OR₂ dans laquelle R₁ représente le reste d'un acide gras comportant de 8 à 29 atomes de carbone, et R₂ représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol; et les esters du pentaérythritol comme le tétraisostéarate de pentaérythrityle,
- les hydrocarbures linéaires ou ramifiés, d'origine minérale ou synthétique, tels que les huiles de paraffine, volatiles ou non, et leurs dérivés, l'isohexadecane, l'isododecane, la vaseline, les polydécènes, le polyisobutène hydrogéné tel que l'huile de Parléam^{®},
- des huiles essentielles naturelles ou synthétiques telles que, par exemple, les huiles d'eucalyptus, de lavandin, de lavande, de vétiver, de litsea cubeba, de citron, de santal, de romarin, de camomille, de sarriette, de noix de muscade, de cannelle, d'hysope, de carvi, d'orange, de géraniol, de cade et de bergamote,
- les alcools gras ayant de 8 à 26 atomes de carbone, comme l'alcool cétylique, l'alcool stéarylique et leur mélange (alcool cétylstéarylique), l'octyl dodécanol, le 2-butyloctanol, le 2-hexyldécanol, le 2-undécylpentadécanol, l'alcool oléique ou l'alcool linoléique,
- les huiles fluorées partiellement hydrocarbonées et/ou siliconées comme celles décrites dans le document JP-A-2-295912,
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que le cyclohexasiloxane et le cyclopentasiloxane ; les polydiméthylsiloxanes comportant des groupements alkyle, alcoxy ou phényle, pendant ou en bout de chaîne siliconée, groupements ayant de 2 à 24 atomes de carbone; les silicones phénylées comme les phényltriméthicones, les phényldiméthicones, les phényltriméthylsiloxydiphényl-siloxancs, les diphényl-diméthicones, les diphénylméthyldiphényl trisiloxanes, les 2-phényléthyttriméthyl-siloxysilicates, et les polyméthylphénylsiloxanes, et
- leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les cires et les acides gras comportant de 8 à 30 atomes de carbone, comme l'acide stéarique, l'acide laurique, l'acide palmitique et l'acide oléique.

A titre de cires pouvant être utilisées selon l'invention, on peut citer les cires d'origine animale telles que la cire d'abeilles, le spermaceti, la cire de lanoline et les dérivés de lanoline, les cires végétales telles que la cire de Carnauba, de Candellila, d'Ouricury, du Japon, le beurre de cacao ou les cires de fibres de liège ou de canne à sucre, les cires minérales, par exemple de paraffine, de vaseline, de lignite ou les cires microcristallines ou les ozokérites, les cires synthétiques parmi lesquelles les cires de polyéthylène, de polytétrafluoroéthylène et les cires obtenues par synthèse de Fisher-Tropsch ou encore les cires de silicone, les huiles hydrogénées concrètes à 25 °C telles que l'huile de ricin hydrogénée, l'huile de jojoba hydrogénée, l'huile de palme hydrogénée, le suif hydrogéné, l'huile de coco hydrogénée et les esters gras concrets à 25°C comme le stéarate d'alkyle en C₂₀-C₄₀ vendu sous la dénomination commerciale « KESTER WAX K82H » par la société KOSTER KEUNEN.

Ces corps gras peuvent être choisis de manière variée par l'homme du métier afin de préparer une composition ayant les propriétés, par exemple de consistance ou de texture, souhaitées.

Les compositions selon l'invention peuvent comprendre une huile volatile.

Par « huile volatile », on entend au sens de l'invention une huile susceptible de s'évaporer au contact des matières kératiniques en moins d'une heure, à température ambiante et pression atmosphérique. Le ou les solvants organiques volatils et les huiles volatiles de l'invention sont des solvants organiques et des huiles cosmétiques volatiles, liquides à température ambiante, ayant une pression de vapeur non nulle, à température ambiante et pression atmosphérique, allant en particulier de 0,13 Pa à 40 000 Pa (10-3 à 300 mm de Hg), en particulier allant de 1,3 Pa à 13 000 Pa (0,01 à 100 mm de Hg), et plus particulièrement allant de 1,3 Pa à 1300 Pa (0,01 à 10 mm de Hg).

On peut citer comme huiles volatiles entre autres, les silicones cycliques ou linéaires renfermant de 2 à 6 atomes de silicium, telles que le cyclohexasiloxane, le dodecamethylpentasiloxane, le decamethyltetrasiloxane, le butyltrisiloxane et l'éthyltrisiloxane. On peut aussi utiliser les hydrocarbures ramifiés tels que par exemple l'isododécane ainsi que les perfluoroalcanes volatiles tels que le dodécafluoropentane et le tétradécafluorohexane, vendus sous les dénominations de « PF 5050^{®} » et « PF 5060^{®}» par la Société 3M et les dérivés de perfluoromorpholine, tels que la 4-trifluorométhyl perfluoromorpholine vendue sous la dénomination « PF 5052^{®} » par la Société 3M.

La quantité de phase huileuse présente dans les compositions selon l'invention peut aller par exemple de 0,01 à 50 % en poids et de préférence de 0,1 à 30 % en poids par rapport au poids total de la composition.

La composition selon l'invention pourra aussi contenir des élastomères de silicone comme les produits commercialisés sous les dénominations « KSG » par la société Shin-Etsu, sous les dénominations « Trefil », « BY29 » ou « EPSX » par la société Dow Corning ou sous les dénominations « Gransil » par la société Grant Industries.

Les compositions selon l'invention peuvent en outre comprendre au moins une matière colorante choisie par exemple parmi les pigments, les nacres, les colorants, les matériaux à effet et leurs mélanges.

Ces matières colorantes peuvent être présentes en une teneur allant de 0,01 % à 50 % en poids, de préférence de 0,01 % à 30 % par rapport au poids total de la composition.

Les compositions selon l'invention peuvent également comprendre une charge notamment en une teneur allant de 0,01 % à 50 % en poids, par rapport au poids total de la composition de préférence allant de 0,01 % à 30 % en poids. Ces charges peuvent être minérales ou organiques de toute forme, plaquettaires, sphériques ou oblongues, quelle que soit la forme cristallographique (par exemple feuillet, cubique, hexagonale, orthorombique, ou amorphe). On peut citer la silice, le talc, le mica, le kaolin, la lauroyl-lysine, l'amidon, le nitrure de bore, les poudres de PTFE, les poudres de PMMA, les poudres de résine de methyl silsesquioxane (comme le Tospearl 145A de GE Silicone), les particules hémisphérique creuse de résine de silicone (comme les NLK 500, NLK 506 et NLK 510 de Takemoto Oil and Fat), le sulfate de Baryum, le carbonate de calcium précipité, le carbonate et l'hydro-carbonate de magnésium, l'hydroxyapatite, les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

La composition selon l'invention peut en outre contenir divers adjuvants couramment utilisés dans le domaine cosmétique, tels que des séquestrants ; des parfums; et des épaississants et gélifiants. Les quantités de ces différents adjuvants et leur nature seront choisies de manière à ne pas nuire aux propriétés de la composition.

Les exemples et figures qui suivent servent à illustrer l'invention sans toutefois présenter un caractère limitatif. Les composés sont, selon le cas, cités en noms chimiques ou en noms CTFA (International Cosmetic Ingrédient Dictionary and Handbook).

### Exemple 1: Crème sous forme d'émulsion Huile/Eau

Cette composition est destinée notamment au soin du visage et du cou, et peut être appliquée quotidiennement.

| Phase A aqueuse: | |
|---|---|
| Eau | QSP 100 |
| % | |
| Glycérine | 5% |
| Adénosine | 0,1 % |
| Conservateur | 0,4 % |
| Acide éthylène diamine tétraacétique, sel disodique | 0,3 % |
| Hyaluronate de Sodium | 0,1 % |
| Polyacrylate de sodium réticulé | 0,5 % |
| Mono-di-palmito-stéarate de sucrose | 1,5% |
| Polymère acide acrylique/acrylate d'alkyles réticulé | 0,2 % |

| Phase B grasse : | |
|---|---|
| Beurre de karité | 2 % |
| Corps gras | 2 % |
| Filtres UV | 15% |
| Acide stéarique | 1,2% |
| Polyacrylate de Stéaryle | 2 % |

| Phase C: | |
|---|---|
| Cyclohexasiloxane | 5 % |
| Polymère réticulé de diméthicone et diméthicone/vinyl diméthicone | 3 % |

| Phase D: | |
|---|---|
| Charges | 1% |

| Phase E: | |
|---|---|
| Rétinol* | 0,07 % |

| | |
|---|---|
| * exprimé en matière active | |

### Mode opératoire:

1 - chauffer A et B séparément à 80 °C jusqu'à homogénéisation complète,
2 - ajouter A à B sous turbine vers 75 °C,
3 - homogénéiser,
4- refroidir à 40 °C et ajouter C sous turbine,
5 - ajouter D puis à 25 °C ajouter E.

### Exemple 2 : Crème sous forme d'émulsion Huile/Eau

Cette composition est destinée notamment au soin du visage et du cou, et peut être appliquée quotidiennement.

| Phase A aqueuse: | |
|---|---|
| Eau | QSP 100% |
| Glycérine | 7 % |
| Hyaluronate de sodiums | 0,08 % |
| Adénosine | 0,1 % |
| Conservateurs | 0,9 % |
| Copolymère AMPS^{®}/méthacrytate de stéaryl éthoxylé réticulé par triméthylolpropane triaacrylate | 0,8% |
| Mono-di-palmito-stéarate de sucrose | 1,5% |
| Copolymère d'acrylates | 0,2 % |
| Polyacrylate de sodium réticulé | 0,8 % |
| Solvant | 3 % |
| Acide Hyaluronique hydrolysé | 0,08 % |

| Phase B grasse : | |
|---|---|
| Beurre de karité | 4 % |
| Polyacrylate de stéaryle | 2 % |
| Acide stéarique | 1,5 % |
| Cire d'abeille blanchie | 1 % |
| Emollients | 10 % |

| Phase C : | |
|---|---|
| Cyclohexasiloxane | 3,5 % |
| Polymère réticulé de diméthicone et diméthicone/vinyl diméthicone | 5 % |

| Phase D: | |
|---|---|
| Charges | 2 % |

| Phase E: | |
|---|---|
| Rétinyl palmitate* | 0,165% |

| Phase F : | |
|---|---|
| Eau de Vichy | 3 % |

| | |
|---|---|
| * exprimé en matière active | |

### Mode opératoire:

1 - chauffer A et B séparément à 80 °C jusqu'à homogénéisation complète,
2 - ajouter A à B sous turbine vers 75 °C,
3 - homogénéiser,
4- refroidir à 40 °C et ajouter C sous turbine,
5 - ajouter D puis à 25°C ajouter E puis F.

### Exemple 3 : Mise en évidence d'une synergie d'action de l'association du rétinol avec l'adénosine

Les effets de l'association sont évalués vis-à-vis de la différenciation et de la prolifération épidermique dans le modèle d'épiderme reconstruit Realskin.

### PROTOCOLE

Les épidermes Réalskin ont été incubés en présence ou en absence d'actifs à partir de J 20 :
Rétinol à 250µM
Adénosine à 10µM
Rétinol 250µM + Adénosine 10µM

Les épidermes sont analysés histologiquement et par immunomarquage Filaggrine (marqueur de différenciation épidermique) à partir de J 27.

La filaggrine, marqueur de différenciation tardive, est un produit de dégradation de la profilaggrine, phosphoprotéine de haut PM contenue dans les granules de kératohyaline. Au moment de la transformation du kératinocyte en cornéocyte, les granules de kératohyaline se désagrègent, la profilaggrine subit une protéolyse séquentielle qui libère des oligomères puis des monomères de filaggrine.

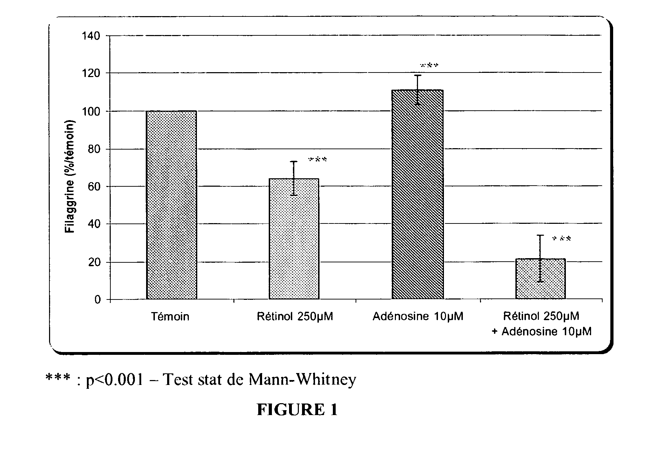

Les résultas sont illustrés en figure 1.

Ils témoignent d'un retard de différenciation sur les peaux traitées au rétinol (-36%); augmentation avec l'adénosine (+11 %) et potentialisation des effets du rétinol avec l'association (-79 %).

Ce test prouve donc l'activité renforcée du rétinol par l'adénosine qui relève à l'évidence d'une synergie dans la mesure où l'effet constaté est significativement supérieur à la somme des effets respectifs du rétinol et de l'adénosine considérés séparément l'un de l'autre.

### Exemple 4: Mise en évidence de l'efficacité d'une composition conforme à l'invention

Cette efficacité a été appréciée selon deux méthodologies à savoir une étude cosmétoclinique et une caractérisation par chromasphère.

### 1. La cosmétoclinique

La composition conforme à celle de l'exemple 1, mais exempte d'EDTA, a été testée sur 50 femmes d'origine caucasienne âgées de 45 à 65 ans, ayant des rides/ridules de la patte d'oie, du front, du sillon nasogénien, inter sourcils, ptose du bas du visage et des problèmes de tonicité cutanée

L'application de la composition a été effectuée 1 fois par jour pendant 8 semaines.

### Résultats : scorage clinique

Le dermatologue a évalué à T0, T28 et T56 jours à l'aide des atlas renseignant sur l'état des rides considérées ci-après, à différents stades de vieillissement cutané:
- les rides du front,
- les rides sous l'oeil,
- les rides du sillon nasogénien,
- la ptose du bas du visage, et
- les rides de la patte d'oie (grade d'inclusion > 3)

Les résultats de l'évolution de l'état des rides considérées en fonction du temps sont rassemblés dans le tableau 1 suivant.

**Tableau 1**

| | **T28jours/T0** | **T56jours/T0** |
|---|---|---|
| **Rides du front** | -17 % S* | -22 % S |
| **Rides sous l'oeil** | -23 % S | -35 % S |
| **Rides du sillon nasogénien** | -11 % S | -29 % S |
| **Ptose du bas du visage** | -10% S | -18% S |
| **Rides de la patte d'oie** | -15% S | -26 % S |

| | | |
|---|---|---|
| *S= significatif | | |

Ces résultats mettent en évidence l'effet significatif du produit sur la diminution de tous les paramètres étudiés après 28 jours d'utilisation quotidienne. L'efficacité est encore renforcée après 56 jours.

### 2. Chromasphère

La Chromasphère est une technique permettant l'acquisition de photos couleur calibrées en utilisant une illumination type lumière du jour (D65), diffuse, homogène et reproductible. Associée à une camera Hitachi HVF22F et à une mire couleur, elle est l'outil de référence pour la mesure de couleur. Elle est notamment décrite dans le document
FR 2 929 344.
- Protocole:: formule testée : composition de l'exemple 1, exempte d'EDTA
- Methodes:: Chromasphère (lumière diffuse) :
- Paramètres:: Sur les images de Chromasphère (Visibilité): Profils Ra (Profondeur moyenne du profil avec Visibility) Profils Rz (Profondeur maximale du profil avec Visibility) Histogrammes: Histos (Aire Tᵢ- T₀ avec Vistogrammes)
- Shéma experimental:: Pas de randomisation T₀: 2 côtés non-traités Tᵢₘₘ: côté gauche non-traité (G)/côté droit traité (D) T2ₘₒᵢₛ: 2 côtés traités

- Application:: Quotidienne pendant 2 mois
- Panel:: 34 modèles de + de 55 ans (Moyenne panel : 66,4 ± 6,3 ans)
- Zone :: Patte d'oie, Inclusion avec score >3.3 sur l'atlas des rides de la patte d'oie
- Temps :: T₀, Tᵢₘₘ, T₂ mois

Concernant les données obtenues grâce aux différentes analyses, elles rendent compte de la répartition APPARENTE des rides (VISIBILITE des rides). Les valeurs sont calculés à partir de photos couleur 2D (.TIFF) acquises en lumière diffuse dans des conditions calibrées et reproductibles (Chromasphère traditionnelle).

La visibilité des rides a été évaluée sur les photos acquises sur la chromasphère traditionnelle à partir du logiciel d'analyse « Visibility ».

Le logiciel « Visibility » permet d'accéder, après avoir transformé l'information couleur en niveaux de gris, aux paramètres de rugosité apparente moyen (Ra) et maximal (Rz) de 5 bandes parallèles verticales réparties sur le champ de l'image, du coin de l'oeil vers la tempe. En plus de ces valeurs, les moyennes sur l'ensemble des 5 bandes sont également calculées.

Les résultats sont présentés dans les tableaux 2 et 3 ci-après:

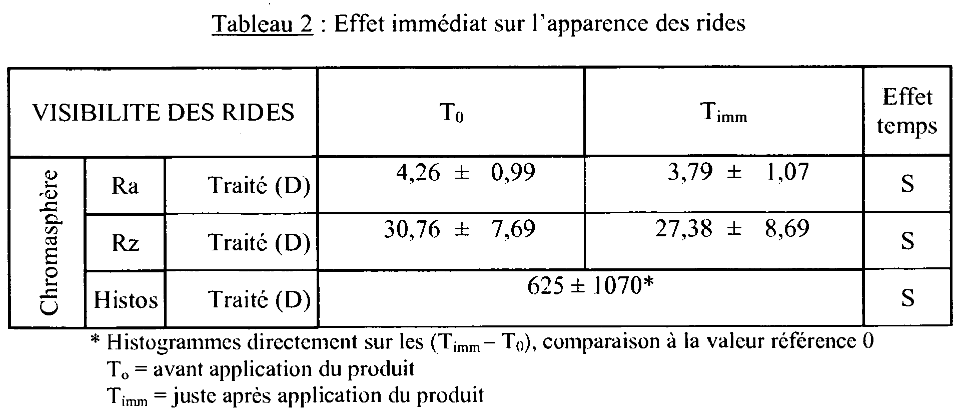

Sur la zone traitée, la formule testée induit un effet immédiat significatif sur la visibilité des rides en chromasphère. Cet effet est mesuré sur le relief le plus profond (Rz) et sur l'ensemble des reliefs analysés (Ra). Un effet significatif est également détecté sur la différence des aires en histogrammes comparée à la valeur référence « 0 » (Histos), signifiant que les variations de niveaux de gris autour du plan moyen ont diminué entre T₀ et Tᵢₘₘ.

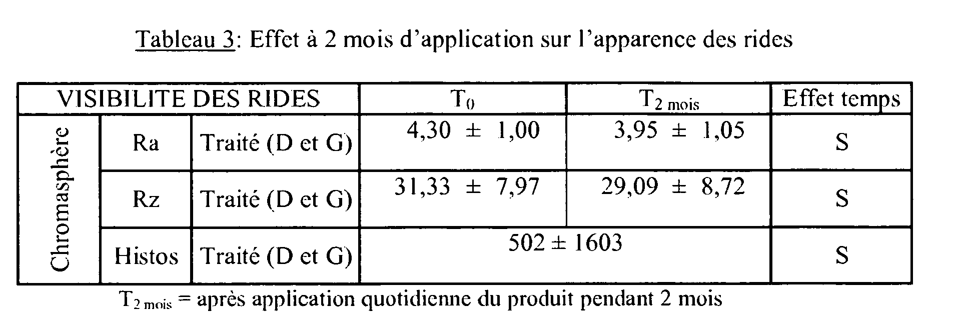

Concernant l'effet long terme de la formule testée, une diminution significative de la visibilité des rides est détectée sur le paramètre de rugosité maximale Rz, de rugosité moyenne Ra et d'histogrammes Histos entre T₀ et T₂ₘₒᵢₛ

### Exemples 5 et 6 : Influence du polymère semi-cristallin à chaîne(s) latérale(s) cristallisable(s) sur l'efficacité anti-rides des compositions

Les exemples suivants de formulations de crèmes sous forme d'émulsion H/E permettent de comparer l'influence du polymère semi-cristallin à chaîne(s) latérale(s) cristallisable(s) sur l'efficacité anti-rides des compositions.

| **Composés** | **Exemple 5 (Invention)** | **Exemple 6 (Comparatif)** |
|---|---|---|
| Phase A aqueuse: | | |
| Eau | QSP 100 % | QSP 100 % |
| Glycérine | 5 % | 5 % |
| Adénosine*** | 0,1 % | 0,1 % |
| Conservateurs | 0,65 % | 0,65 % |
| Acide éthylène diamine tétraacétique, sel disodique | 0,3 % | 0,3 % |
| Mono-di-palmito-stéarate de sucrose | 1,5 % | 1,5 % |
| Polymère acide acrylique/acrylate d'alkyles réticulé | 0,2 % | 0,2 % |

| Phase B1 grasse: | | |
|---|---|---|
| Corps gras | 5% | 5% |
| Filtres UV | 15% | 15% |
| Acide stéarique | 1,2% | 1,2% |
| Caprylyl glycol | 0,2 % | 0,2% |
| Polyacrylate de Stéaryle** | 2 % | - |

| Phase B2: | | |
|---|---|---|
| Parfum | 0,4% | 0,4% |

| Phase C1: | | |
|---|---|---|
| Cyclohexasiloxane | 3 % | 3 % |
| Hyaluronate de Sodium | 0,1% | 0,1 % |
| Polyacrylate de sodium réticulé | 0,5 % | 0,5% |

| Phase C2 : | | |
|---|---|---|
| Cyclohexasiloxane | 2 % | 2 % |
| Polymère réticulé de diméthicone et diméthicone/vinyl diméthicone | 3 % | 3 % |

| Phase D: | | |
|---|---|---|
| Charge | 1% | 1% |

| Phase E: | | |
|---|---|---|
| Rétinol* | 0,07% | 0,07% |

| | | |
|---|---|---|
| * Rétinol 10 SU commercialisé par BASF (exprimé en matière active) ** Intelimer^{®}IPA 13-1 de la Société AIR PRODUCT and CHEMICALS *** Adénosine commercialisée par PHARMA WALDHOF GmbH | | |

### Mode opératoire :

1- chauffer A et B1 séparément à 80 °C jusqu'à homogénéisation complète,
2- ajouter A à B1 + B2 sous turbine vers 75 °C,
3- homogénéiser, ajouter C1 à 40°C sous turbine,
4- puis C2 à 30°C,
5- ajouter D puis à 25 °C, ajouter E sous inertage d'azote.

### Evaluation :

Les crèmes obtenues sont évaluées sur un panel de 6 femmes quant aux effets cosmétiques immédiats observés.

Au jour J, une quantité de 0,30 mL de la composition de l'exemple 5 selon l'invention, est appliquée sur un demi-visage de chacune des six femmes, en particulier dans la zone située au-dessous de l'oeil.

Une photographie est réalisée juste après l'application.

Le lendemain (J+1), une quantité de 0,30 mL de la composition de l'exemple comparatif 6 est appliquée sur le même demi-visage qu'au jour J, pour chacune des six femmes.

Une photographie est réalisée juste après l'application.

L'application des compositions sur les demi-visages est randomisée: autrement dit, pour la première personne, le demi-visage choisi est le demi-visage droit, pour la deuxième personne, le demi-visage choisi est le demi-visage gauche, etc.

Les photographies prises respectivement après application de la composition de l'exemple 5 et de la composition de l'exemple 6 sur les demi-visages sont comparées.

### Résultats

Des résultats de l'étude, il est observé, après application de la crème de l'exemple 5 selon l'invention, une atténuation des rides sous l'oeil. Celles-ci apparaissent beaucoup moins prononcées. Une telle atténuation des rides sous l'oeil n'est pas observée après application de la crème de l'exemple 6 hors invention.

Ainsi, la présence du polymère semi-cristallin à chaîne(s) latérale(s) cristallisable(s) permet de renforcer significativement l'efficacité anti-rides des compositions selon l'invention, au regard d'une composition qui en serait dépourvue.

## Revendications

1. Composition cosmétique ou dermatologique, **caractérisée par le fait qu'**elle comprend, dans un milieu physiologiquement acceptable, une association contenant au moins un rétinoïde , un composé non-phosphaté à base d'adénosine et un polymère semi-cristallin à chaîne(s) latérale(s) cristallisable(s).

2. Composition selon la revendication précédente dans laquelle ledit rétinoide est choisi parmi le rétinol, le rétinal, l'acide rétinoïque, un ester de rétinol et d'un acide en C₂-C₂₀, tel que le propionate, l'acétate, le linoléate, ou le palmitate de rétinol.

3. Composition selon l'une quelconque des revendications précédentes, **caractérisée en ce qu'**elle comprend de 0,005 à 5 % en poids, de préférence de 0,01 à 2 % en poids, et notamment de 0,05 à 0,5 % en poids de rétinoïde par rapport au poids total de la composition.

4. Composition selon l'une quelconque des revendications précédentes, **caractérisée en ce que** le composé non-phosphaté à base d'adénosine est l'adénosine.

5. Composition selon l'une quelconque des revendications précédentes, **caractérisée en ce qu'**elle comprend de 0,0001 à 5 % en poids, de préférence de 0,001 à 1% en poids, et notamment de 0,01 à 0,5 % en poids de composé non-phosphaté à base d'adénosine par rapport au poids total de la composition.

6. Composition selon l'une quelconque des revendications précédentes, dans laquelle le polymère semi-cristallin à chaîne(s) latérale(s) cristallisable(s) est choisi parmi les homopolymères résultant de la polymérisation d'au moins un monomère à chaîne latérale cristallisable choisis parmi les (méth)acrylates d'alkyle saturés en C₁₀ à C₃₀, qui peut être représenté par la formule suivante : 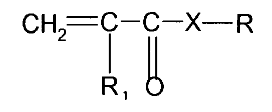 dans laquelle R₁ est H ou CH₃, R représente un groupe alkyle en C₁₀ à C₃₀, et X représente O.

7. Composition selon l'une quelconque des revendications précédentes, dans laquelle le polymère semi-cristallin à chaîne(s) latérale(s) cristallisable(s) est choisi parmi les homopolymères résultant de la polymérisation d'un monomère à chaîne cristallisable choisi parmi les acrylates d'alkyle en C₁₄-C₂₄ et les méthacrylates d'alkyle en C₁₄-C₂₄.

8. Composition selon l'une quelconque des revendications précédentes, dans laquelle le polymère semi-cristallin à chaîne(s) latérale(s) cristallisable(s) est choisi parmi l'homopolymére d'acrylate de stéaryle et l'homopolymère d'acrylate de béhényle.

9. Composition selon l'une quelconque des revendications précédentes, **caractérisée en ce qu'**elle comprend au moins 0,6 % en poids, en particulier au moins 1% en poids, plus particulièrement de 1 à 5 %, en particulier de 1 à 3 % en poids de polymère semi-cristallin à chaîne(s) latérale(s) cristallisable(s) par rapport au poids total de la composition.

10. Composition selon l'une quelconque des revendications précédentes, **caractérisée en ce qu'**elle comprend du rétinol ou du palmitate de rétinol, en association avec un composé non-phosphaté à base d'adénosine et un homopolymère de (méth)acrylates d'alkyle en C₁₀-C₃₀, en particulier le polyacrylate de stéaryle.

11. Composition selon l'une quelconque des revendications précédentes, **caractérisée en ce qu'**elle comprend en outre au moins un polymère additionnel choisi parmi les polymères acryliques, les polysaccharides modifiés hydrophobes et les esters d'acide gras et de polyols.

12. Composition selon l'une quelconque des revendications précédentes, **caractérisée en ce qu'**elle comprend au moins un actif cosmétique complémentaire choisi parmi des actifs anti-rides autres que l'adénosine, le rétinol et ses dérivés, les filtres UV, les agents desquamants, les agents antioxydants, les agents hydratants, les actifs stimulants la synthèse des macromolécules dermiques et/ou épidermiques, les agents dermodécontractants et leurs mélanges.

13. Utilisation cosmétique d'une association comprenant au moins un rétinoïde , un composé non-phosphaté à base d'adénosine et un polymère semi-cristallin à chaîne(s) latérale(s) cristallisable(s) pour prévenir et/ou traiter les signes de vieillissement cutané, et/ou pour prévenir et/ou traiter la peau ridée et/ou la peau relâchée.

14. Utilisation selon la revendication 13, pour diminuer et/ou effacer les imperfections cutanés, notamment les rides, en particulier les rides embryonnaires, profondes et/ou réversibles, et plus particulièrement les rides disposées radialement autour de la bouche et/ou des yeux, en particulier les rides de la patte d'oie, les rides sous l'oeil et/ou situées au niveau du front, en particulier la ride dite du lion, située au niveau de la gabelle, dans l'espace inter-sourcillier, et/ou disposées horizontalement sur le front, les rides du sillon nasogénien, et la ptôse du bas du visage, et/ou pour obtenir une peau plus lisse, plus homogène, plus ferme, tonique et élastique.

15. Procédé de traitement non thérapeutique ou cosmétique de la peau, notamment humaine, destiné à prévenir et/ou traiter les signes cutanés du vieillissement, notamment les rides, comprenant l'application sur la peau d'une association et/ou d'une composition telles que définies selon l'une quelconque des revendications 1 à 12.

## Claims

1. Cosmetic or dermatological composition, comprising, in a physiologically acceptable medium, a combination containing at least one retinoid, one adenosine-based nonphosphated compound and one semicrystalline polymer with one or more crystallizable side chain(s).

2. Composition according to the previous claim, wherein said retinoid is chosen from retinol, retinal, retinoic acid, an ester of retinol and of a C₂-C₂₀ acid, such as propionate, acetate, linoleate or retinol palmitate.

3. Composition according to any one of the previous claims, comprising from 0.005% to 5% by weight, preferably from 0.01 to 2% by weight and in particular from 0.05 to 0.5 % by weight of retinoid, relative to the total weight of the composition.

4. Composition according to any one of the previous claims, wherein the adenosine-based nonphosphated compound is adenosine.

5. Composition according to any one of the previous claims, comprising from 0.0001% to 5% by weight, preferably from 0.001 to 1% by weight and in particular from 0.01 to 0.5% by weight of adenosine-based nonphosphated compound, relative to the total weight of the composition.

6. Composition according to any one of the previous claims, wherein the semicrystalline polymer with one or more crystallizable side chain(s) is chosen from the homopolymers resulting from the polymerization of at least one monomer with a crystallizable side chain, chosen from saturated C₁₀ to C₃₀ alkyl (meth)acrylates, which may be represented by the formula below: 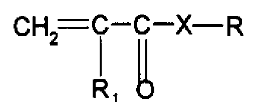 in which R₁ is H or CH₃, R represents a C₁₀ to C₃₀ alkyl group, and X represents O.

7. Composition according to any one of the previous claims, wherein the semicrystalline polymer with one or more crystallizable side chain(s) is chosen from homopolymers resulting from the polymerization of a monomer with a crystallizable chain, chosen from C₁₄-C₂₄ alkyl acrylates and C₁₄-C₂₄ alkyl methacrylates.

8. Composition according to any one of the previous claims, wherein the semicrystalline polymer with one or more crystallizable side chain(s) is chosen from the homopolymer of stearyl acrylate and the homopolymer of behenyl acrylate.

9. Composition according to any one of the previous claims, comprising at least 0.6% by weight, in particular at least 1% by weight, more particularly from 1 to 5% by weight, in particular from 1 to 3% by weight, of semicrystalline polymer with one or more crystallizable side chain(s), relative to the total weight of the composition.

10. Composition according to any one of the previous claims, comprising retinol or retinyl palmitate, in combination with an adenosine-based nonphosphated compound and a C₁₀-C₃₀ alkyl (meth)acrylate homopolymer, in particular the stearyl polyacrylate.

11. Composition according to any one of the previous claims, further comprising at least one additional polymer chosen from acrylic polymers, hydrophobically modified polysaccharides and fatty acid esters of polyols.

12. Composition according to any one of the previous claims, comprising at least one supplementary cosmetic active agent chosen from antiwrinkle active agents other than adenosine, retinol and derivatives thereof, UV screening agents, desquamating agents, antioxidants, moisturizers, active agents for stimulating the synthesis of dermal and/or epidermal macromolecules, dermodecontracting agents, and mixtures thereof.

13. Cosmetic use of a combination comprising at least one retinoid, one adenosine-based nonphosphated compound and one semicrystalline polymer with one or more crystallizable side chain(s) for preventing and/or treating the signs of skin ageing and/or for preventing and/or treating wrinkled skin and/or sagging skin.

14. Use according to claims 13, for reducing and/or smoothing out skin imperfections, in particular wrinkles, more particularly embryonic, deep or reversible wrinkles, and more particularly the wrinkles lying radially around the mouth and/or the eyes, in particular crowsfeet wrinkles, wrinkles under the eye and/or on the forehead, in particular the "lion" wrinkle, located on the glabella, in the space between the eyebrows, and/or lying horizontally on the forehead, wrinkles of the nasal groove, and ptosis of the lower part of the face, and/or for obtaining skin which is smoother, more homogeneous, firmer, more tonic and more elastic.

15. Nontherapeutic or cosmetic treatment method for the skin in particular human skin, intended to prevent and/or treat the cutaneous signs of ageing, in particular wrinkles, comprising the application, to the skin, of a combination and/or of a composition as defined according to any one of claims 1 to 12.

## Patentansprüche

1. Kosmetische oder dermatologische Zusammensetzung, **dadurch gekennzeichnet, dass** sie in einem physiologisch verträglichen Medium eine Kombination enthält, die mindestens ein Retinoid, eine nicht-phosphatierte Verbindung auf der Basis von Adenosin und ein sennikristallines Polymer mit kristallisierbarer(n) Seitenkette(n) enthält.

2. Zusammensetzung nach dem vorhergehenden Anspruch, wobei das Retinoid ausgewählt ist aus Retinol, Retinal, Retinsäure, einem Ester von Retinol und einer C₂-C₂₀-Säure, wie Retinolpropionat, -acetat, -linoleat oder -palmitat.

3. Zusammensetzung nach einem der vorhergehenden Ansprüche, **dadurch gekennzeichnet, dass** sie von 0,005 bis 5 Gew.-%, vorzugsweise von 0,01 bis 2 Gew.-% und insbesondere von 0,05 bis 0,5 Gew.-% Retionoid umfasst, bezogen auf das Gesamtgewicht der Zusammensetzung.

4. Zusammensetzung nach einem der vorhergehenden Ansprüche, **dadurch gekennzeichnet, dass** die nicht-phosphatierte Verbindung auf der Basis von Adenosin Adenosin ist.

5. Zusammensetzung nach einem der vorhergehenden Ansprüche, **dadurch gekennzeichnet, dass** sie von 0,0001 bis 5 Gew.-%, vorzugsweise von 0,001 bis 1 Gew.-% und insbesondere von 0,01 bis 0,5 Gew.-% nicht phosphatierte Verbindung auf der Basis von Adenosin, bezogen auf das Gesamtgewicht der Zusammensetzung, enthält.

6. Zusammensetzung nach einem der vorhergehenden Ansprüche, wobei das semikristalline Polymer mit kristalliserbarer(n) Seitenkette(n) ausgewählt ist aus Homopolymeren, die aus der Polymerisation von mindestens einem Monomer mit kristallisierbarer Seitenkette, ausgewählt aus gesättigten C₁₀- bis C₃₀-Alkyl(meth)acrylaten hervorgehen, die durch die folgende Formel dargestellt werden können: 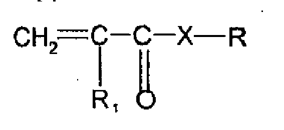 wobei R₁ H oder CH₃ ist, R eine C₁₀- bis C₃₀-Alkylgruppe darstellt und X O darstellt.

7. Zusammensetzung nach einem der vorhergehenden Ansprüche, wobei das semikristalline Polymer mit kristallisierbarer(n) Seitenkette(n) ausgewählt ist aus Homopolymeren, die aus der Polymerisation von einem Monomer mit kristallisierbarer Kette, ausgewählt aus C₁₄-C₂₄-Alkylacrylaten und C₁₄-C₂₄-Alkylmethacrylaten, hervorgehen.

8. Zusammensetzung nach einem der vorhergehenden Ansprüche, wobei das semikristalline Polymer mit kristallisierbare(n)r Seitenkette(n) ausgewählt ist aus Stearylacrylat-Homopolymer und Behenylacrylat-Homopolymer.

9. Zusammensetzung nach einem der vorhergehenden Ansprüche, **dadurch gekennzeichnet, dass** sie mindestens 0,6 Gew.-%, insbesondere mindestens 1 Gew.-%, spezieller von 1 bis 5 Gew.-%, insbesondere von 1 bis 3 Gew.-% semikristallines Polymer mit kristallisierbare(n)r Seitenkette(n), bezogen auf das Gesamtgewicht der Zusammensetzung, umfasst.

10. Zusammensetzung nach einem der vorhergehenden Ansprüche, **dadurch gekennzeichnet, dass** sie das Retinol oder Retinolpalmitat in Kombination mit einer nichtphosphatierten Verbindung auf der Basis von Adenosin und einem Homopolymer von C₁₀-C₃₀-Alkyl(meth)acrylaten, insbesondere Stearylpolyacrylat, umfasst.

11. Zusammensetzung nach einem der vorhergehenden Ansprüche, **dadurch gekennzeichnet, dass** es weiterhin mindestens ein zusätzliches Polymer umfasst, ausgewählt aus Acrylpolymeren, hydrophoben modifizierten Polysacchariden und Estern von Fettsäure und Polyolen.

12. Zusammensetzung nach einem der vorhergehenden Ansprüche, **dadurch gekennzeichnet, dass** es mindestens einen zusätzlichen kosmetischen Wirkstoff umfasst, ausgewählt aus Antifalteriwirkstoffen, die anders sind als Adenosin, Retinol und seine Derivate, UV-Filtern, Desquamationsmitteln, Antioxidantien, Hydratationsmitteln, Wirkstoffen, die die Synthese von Makromolekülen der Lederhaut oder Epidermis stimulieren, Haut-Dekontraktationsmittel und ihren Gemischen.

13. Kosmetische Verwendung einer Kombination, umfassend mindestens ein Retinoid, eine nicht-phosphatierte Verbindung auf der Basis von Adenosin und ein semikristallines Polymer mit kristalliserbarer(n) Seitenkette(n) zur Vorbeugung und/oder Behandlung der Haut-Alterungsanzeichen, und/oder zur Vorbeugung und/oder Behandlung von faltiger Haut und/oder schlaffer Haut.

14. Verwendung nach Anspruch 13, zur Verminderung und/oder Beseitigung von Haut-Unzulänglichkeiten, insbesondere von Falten, insbesondere von tiefen und/oder reversiblen Embryonalfalten, und insbesondere von Falten, die radial um den Mund und/oder die Augen liegen, insbesondere von Krähenfüßen, Falten unter dem Auge und/oder auf der Stirn, insbesondere die Zornesfalte genannte Falte auf der Glabella, im Bereich zwischen den Augenbrauen, und/oder horizontal auf der Stirn, Nasolabialfalten, Ptosis der unteren Gesichtshälfte, und/oder zum Erhalt einer glatteren, homogeneren, festeren, tonischen und elastischen Haut.

15. Verfahren zur nicht therapeutischen oder kosmetischen Behandlung der Haut, insbesondere menschlicher Haut, das zur Vorbeugung und/oder Behandlung der Haut-Alterungsanzeichen, insbesondere von Falten, bestimmt ist, umfassend das Aufbringen einer Kombination und/oder einer Zusammensetzung wie nach einem der Ansprüche 1 bis 12 definiert auf die Haut.
